# EUROPÄISCHE PATENTSCHRIFT

(11) **EP 3 942 091 B1**
(45) Veröffentlichungstag und Bekanntmachung des Hinweises auf die Patenterteilung: **16.11.2022**
(21) Anmeldenummer: 21716406.0
(22) Anmeldetag: 01.04.2021
(51) Int. Cl.: C25F 3/24, C25F 3/06, C23C 22/50, B24C 1/06, B23P 15/00, A61B 17/00, B24C 11/00

(54) **VERFAHREN ZUM OBERFLÄCHENBEHANDELN UND/ODER HERSTELLEN EINES MEDIZINTECHNISCHEN PRODUKTS SOWIE EIN MEDIZINTECHNISCHES PRODUKT**
METHOD FOR THE SURFACE TREATMENT AND/OR MANUFACTURE OF A MEDICAL PRODUCT, AND MEDICAL PRODUCT
PROCÉDÉ DE TRAITEMENT DE SURFACE ET/OU DE FABRICATION D'UN PRODUIT MÉDICAL, ET PRODUIT MÉDICAL

(30) Priorität: 06.04.2020 DE 102020204430
(43) Veröffentlichungstag der Anmeldung: 26.01.2022
(73) Patentinhaber: Aesculap AG, 78532 Tuttlingen (DE)
(72) Erfinder: WAIDELICH, Lukas, 78532 Tuttlingen (DE); GASSNER, Andreas, 78532 Tuttlingen (DE); SCHAUER, Ramon, 78532 Tuttlingen (DE); GRIMM, Christian, 78532 Tuttlingen (DE); BONER, Stefan, 72517 Sigmaringendorf (DE)
(74) Vertreter: Patentanwälte Ruff, Wilhelm, Beier, Dauster & Partner mbB
(86) Internationale Anmeldenummer: PCT/EP2021/058713
(87) Internationale Veröffentlichungsnummer: WO 2021/204699

(56) Entgegenhaltungen:
- WO-A1-2005/004941
- WO-A1-2017/151548
- CN-B- 106 048 707

## Beschreibung

### ANWENDUNGSGEBIET UND STAND DER TECHNIK

Die Erfindung betrifft ein Verfahren zum Oberflächenbehandeln oder -bearbeiten und/oder Herstellen eines medizintechnischen Produkts sowie ein medizintechnisches Produkt.

Medizintechnische Produkte, wie insbesondere chirurgische Instrumente, werden vor ihrer Fertigstellung in der Regel einer Oberflächenbehandlung unterworfen. Hierzu können die Oberflächen der Produkte beispielsweise mittels Gleit- und/oder Bandschleifen bearbeitet werden. Dadurch können Fehler im Vormaterial und/oder schmiedebedingte Fehler, wie beispielsweise entkohlte Bereiche, oder Oberflächenfehler, wie beispielsweise Poren, Narben oder Risse, beseitigt werden, welche sich anderenfalls nachteilig auf die Korrosionsbeständigkeit der Produkte auswirken würden.

Allerdings können durch Bandschleifen feine Kerben oder Anhebungen auf der Produktoberfläche entstehen. Diese können bei einem nachfolgenden Behandlungsschritt umgebogen oder eingedrückt werden. Dadurch können Materialdopplungen entstehen. Außerdem kann es zu einem vereinzelten Materialübertrag, beispielsweise von Siliziumoxidpartikeln, von einem Schleifband auf die Produktoberfläche kommen. Ein derartiger Materialübertrag und die mit der mechanischen Bearbeitung einhergehende Belastung des medizintechnischen Produkts können wiederum Eigenspannungen im Produkt erzeugen oder erhöhen. Problematisch ist zudem, dass beim Schleifen nicht beseitigte oder erzeugte Oberflächenfehler des Produkts in einem nachfolgenden Behandlungsschritt nur noch begrenzt beseitigt werden können.

Zur Mattierung eines medizintechnischen Produkts können sphärische Strahlmittel, wie beispielsweise Glasperlen, verwendet werden. Dadurch kommt es zu einer plastischen Verformung der Produktoberfläche, wodurch diese vergrößert und aufgeraut wird. Da Glasperlen im Allgemeinen sehr hart (Härte 6 Mohs) und obendrein spröde sind, kommt es im Laufe der Zeit zu einem teilweisen Bruch des Strahlmittels. Dadurch treffen während des Mattierungsschrittes sowohl sphärische Glasperlen als auch gebrochene Glasperlen auf die Oberfläche des Produkts. Während gebrochene Glasperlen scharfe Kerben auf der Produktoberfläche erzeugen, hinterlassen ungebrochene Glasperlen sphärische Eindrücke auf der Oberfläche des Produkts. Durch das Auftreffen von gebrochenen und ungebrochenen Glasperlen kommt es zu einer Wechselwirkung zwischen der durch die gebrochenen Glasperlen eingekerbten Produktoberfläche und der durch die ungebrochenen Glasperlen eingeglätteten Produktoberfläche. Hierdurch können ebenfalls Materialdopplungen entstehen. Zusätzlich zur plastischen Verformung und der hiermit einhergehenden Erzeugung von Eigenspannungen kann ein Materialübertrag des Strahlmittels auf die Produktoberfläche stattfinden. Dieser Materialübertrag ist besonders stark im Bereich von Kerben, in welchen Materialanhäufungen der Glasperlen zurückbleiben können.

Alternativ zu der vorstehend am Beispiel von Glasperlen beschriebenen Strahlmittelbehandlung können die Oberflächen von medizintechnischen Produkten gebürstet werden. Hierzu können die Produktoberflächen mit Bürstscheiben, beispielsweise mit Hilfe eines scheibenförmig gestalteten Schleifvlieses oder von scheibenförmig angeordneten Nylonfasern mit Schleifpartikeln, bearbeitet werden. Auf den Bürstscheiben sind üblicherweise Aluminium- und/oder Siliziumoxidpartikel aufgebracht. Durch einen Bürstschritt erhöht sich zwar die Korrosionsbeständigkeit der Produktoberfläche im Vergleich zu einer mattierten Produktoberfläche, nachteilig ist jedoch, dass gebürstete Produktoberflächen ein stärkeres Reflexionsverhalten aufweisen als mattierte Produktoberflächen.

Es ist ferner bekannt, dass sich durch Materialdopplungen ausgebildete Mikrostrukturen oder Kerben auf einer Produktoberfläche sowie eine damit einhergehende Erzeugung oder Erhöhung von Eigenspannungen im Produkt nachteilig auf dessen Korrosionsbeständigkeit auswirken. Im Falle eines Materialübertrags, beispielsweise während eines Bandschleifens und/oder Mattierens, kommt hinzu, dass das übertragene Material zusätzliche Mikrostrukturen generiert sowie die Schwächung einer Passivierungsschicht bewirken kann.

Aus der WO 2017/151548 A1, CN 106 048 707 B sowie WO 2005/004941 A1 ist jeweils ein Verfahren zum Herstellen eines medizintechnischen Produkts bekannt, bei dem eine Oberfläche des medizintechnischen Produkts mit einem Strahlmittel mattiert und die mattierte Oberfläche des medizintechnischen Produkts anschließend elektropoliert wird.

### AUFGABE UND LÖSUNG

Der Erfindung liegt die Aufgabe zugrunde, ein Verfahren zum Oberflächenbehandeln oder -bearbeiten und/oder Herstellen eines medizintechnischen Produkts bereitzustellen, welches bei gattungsgemäßen Verfahren auftretende Nachteile wenigstens teilweise vermeidet und insbesondere zu einem medizintechnischen Produkt mit erhöhter Korrosions- und Kratzbeständigkeit sowie verringertem Reflexionsverhalten führt.

Die Erfindung stellt sich des Weiteren die Aufgabe, ein entsprechendes medizintechnisches Produkt bereitzustellen.

Die oben genannten Aufgaben werden erfindungsgemäß gelöst durch ein Verfahren mit den Merkmalen des unabhängigen Anspruchs 1 sowie durch ein medizintechnisches Produkt gemäß Anspruch 14. Bevorzugte Ausgestaltungen des Verfahrens und des medizintechnischen Produkts sind Gegenstand der abhängigen Ansprüche sowie der Beschreibung. Der Wortlaut sämtlicher Ansprüche wird hiermit durch ausdrückliche Bezugnahme zum Inhalt der Beschreibung gemacht.

Gemäß einem ersten Aspekt betrifft die Erfindung ein Verfahren zum Oberflächenbehandeln oder -bearbeiten und/oder Herstellen eines medizintechnischen Produkts, wobei das medizintechnische Produkt ein Metall oder eine Legierung aufweist oder aus einem Metall oder einer Legierung besteht. Das Verfahren weist die folgenden Schritte auf:
a) Mattieren einer Oberfläche des medizintechnischen Produkts,
b) Elektropolieren der mattierten Oberfläche des medizintechnischen Produkts,
c) elektrochemisches Ätzen der mattierten und elektropolierten Oberfläche des medizintechnischen Produkts und
d) Elektropolieren der mattierten, elektropolierten und elektrochemisch geätzten Oberfläche des medizintechnischen Produkts.

Das Verfahren kann die vorgenannten Schritte insbesondere unmittelbar aufeinanderfolgend aufweisen.

Der Ausdruck "medizintechnisches Produkt" kann im Sinne der vorliegenden Erfindung ein medizintechnisches Endprodukt, vorzugsweise ein chirurgisches Instrument, oder eine Vorstufe, insbesondere ein Halbzeug, ein Rohling oder ein Halbfabrikat, eines medizintechnischen Endprodukts, vorzugsweise eines chirurgischen Instruments, oder ein Bauteil eines medizintechnischen Endprodukts, vorzugsweise eines chirurgischen Instruments, bedeuten.

Unter dem Ausdruck "Legierung" soll im Sinne der vorliegenden Erfindung ein makroskopisch homogener metallischer Werkstoff aus wenigstens zwei Elementen (Komponenten) verstanden werden, von welchen wenigstens ein Element ein Metall ist. Demnach kann der Ausdruck "Legierung" im Sinne der vorliegenden Erfindung einen makroskopisch homogenen metallischen Werkstoff bedeuten, welcher aus wenigstens zwei verschiedenen Metallen besteht. Alternativ kann der Ausdruck "Legierung" im Sinne der vorliegenden Erfindung einen makroskopisch homogenen metallischen Werkstoff bedeuten, welcher aus wenigstens einem Metall sowie wenigstens einem Nichtmetall, wie beispielsweise Kohlenstoff, besteht.

Überraschenderweise stellte sich heraus, dass sich die eingangs im Zusammenhang von konventionellen Oberflächenbehandlungen von medizintechnischen Produkten erwähnten Nachteile durch eine Kombination eines Mattierungsschritts, eines (ersten) Elektropolierschritts, eines elektrochemischen Ätzschrittschritts sowie eines weiteren (zweiten) Elektropolierschritts teilweise oder sogar vollständig vermeiden lassen.

So wird durch den Mattierungsschritt eine die Lichtreflexion verringernde Mikrostruktur auf der Oberfläche des medizintechnischen Produkts erzeugt. Der anschließende Elektropolierschritt bewirkt eine Glättung dieser Mikrostruktur, wodurch eine glatte und glänzende Produktoberfläche erzeugt wird. Zusätzlich führt der Elektropolierschritt durch Adsorption von Sauerstoff vorteilhafterweise zur Ausbildung einer Passivschicht, insbesondere dicken und dichten Passivschicht, auf der elektropolierten Produktoberfläche und mithin zu einer Verbesserung der Korrosionsbeständigkeit des medizintechnischen Produkts. Der nachfolgende elektrochemische Ätzschritt bewirkt mit besonderem Vorteil ebenfalls eine Verbesserung der Korrosionsbeständigkeit, insbesondere eine zusätzliche Verbesserung der Korrosionsbeständigkeit, des medizintechnischen Produkts. Die (zusätzliche) Verbesserung der Korrosionsbeständigkeit beruht insbesondere (ebenfalls) auf einer begünstigten Ausbildung einer Passivschicht auf der Produktoberfläche. Ferner kann die durch den elektrochemischen Ätzschritt bewirkte Verbesserung der Korrosionsbeständigkeit das Ergebnis des Abbaus von korrosionsauslösenden Materialfehlern und/oder der Vermeidung oder Reduktion von Druck- und Zugspannungen und/oder Materialdopplungen und/oder Materialüberlappungen auf der Oberfläche des medizintechnischen Produkts sein. Durch die Ausbildung einer vorzugsweise offenen Mikrostruktur auf der Produktoberfläche, bewirkt der elektrochemische Ätzschritt ferner eine (erneute) Verringerung der Lichtreflexion, welche durch den (ersten) Elektropolierschritt aufgrund der Glättung der durch die Mattierung erzeugten Mikrostruktur leicht erhöht wird. Der elektrochemische Ätzschritt bewirkt somit nicht nur eine Verbesserung der Korrosionsbeständigkeit des medizintechnischen Produkts, sondern auch eine Mattierung von dessen Oberfläche. Dadurch vereinfacht sich die Handhabung des medizintechnischen Produkts für einen Anwender signifikant. Beispielsweise kann hierdurch verhindert werden, dass ein Chirurg im Operationssaal durch das medizintechnische Produkt geblendet wird. Der anschließende weitere (zweite) Elektropolierschritt bewirkt vorzugsweise eine leichte Glättung der Oberfläche des medizintechnischen Produkts, wodurch ein matter Glanz erzeugt wird. Dadurch ist im Ergebnis ein verbessertes Reflexionsverhalten des medizintechnischen Produkts erzielbar, insbesondere ohne die durch den elektrochemisch Ätzschritt erzeugte Mikrostruktur zu glätten.

Im Falle eines medizintechnischen Produkts aus einem chromhaltigen oder chromlegierten Edelstahl beruht der positive Einfluss des elektrochemischen Ätzschritts auf das Lichtreflexionsverhalten sowie die Korrosionsbeständigkeit des medizintechnischen Produkts insbesondere darauf, dass während des elektrochemischen Ätzvorgangs sechswertige Chromionen in Lösung gehen. Dies hat zur Folge, dass chromreiche Oxidschichten an der Oberfläche des medizintechnischen Produkts entfernt werden, wodurch ein direkter Angriff einer für den elektrochemischen Ätzvorgang verwendeten Säure auf chemische und physikalische Inhomogenitäten, wie chromcarbidhaltige Bereiche um Chromcarbide, der Oberfläche des medizintechnischen Produkts ermöglicht wird. Dadurch entsteht, insbesondere an Stellen ehemaliger Chromcarbid-Bereiche, eine Mikrostruktur, insbesondere mit oder in Form von vorzugsweise offenen Ätzgrübchen, auf der Oberfläche des medizintechnischen Produkts. Zusätzlich kann vorteilhafterweise eine Auflösung von Grenzbereichen, insbesondere von Lattenund Subblockgrenzen, erfolgen, die insbesondere ein Herausstechen von einzelnen Martensitlatten zur Folge haben kann. Das Resultat ist eine aufgeraute und insbesondere matt wirkende Produktoberfläche, an welcher einfallendes Licht gestreut werden kann. Durch den Abbau chromverarmter Bereiche auf der Oberfläche des medizintechnischen Produkts wird ferner vorteilhafterweise das Risiko der Entstehung von Keimstellen einer Lochkorrosion gesenkt.

Weitere Vorteile des Verfahrens betreffen eine verbesserte Reinigbarkeit des medizintechnischen Produkts sowie insbesondere eine geringere Neigung des medizintechnischen Produkts zu Oberflächenverfärbungen.

In Ausgestaltung der Erfindung wird vor dem Durchführen von Schritt a) ein Schleifen, vorzugsweise ein Gleit- und/oder Bandschleifen, der Oberfläche des medizintechnischen Produkts durchgeführt.

Zum Gleitschleifen wird das medizintechnische Produkt bevorzugt zusammen mit Gleitschleifkörpern, welche vorzugsweise als Schüttgut gestaltet sind, oder zusammen mit einer Gleitschleifkörper und optional Zusatzstoffe enthaltenden wässrigen Lösung in einen Behälter gegeben. Die optional vorgesehenen Zusatzstoffe können ausgewählt sein aus der Gruppe bestehend aus Korrosionsschutzmittel, Entfettungsmittel, Beizmittel, Trennmittel (beispielsweise Kunststoffkügelchen mit einem Durchmesser < 1 mm) und Mischungen davon. Durch eine solche Lösung lassen sich in vorteilhafter Weise ein durch die Gleitschleifkörper entstehender Abrieb sowie ein Produktabtrag aufnehmen und abtransportieren. Abhängig vom jeweils verwendeten Zusatzstoff lassen sich darüber hinaus weitere Effekte realisieren, wie beispielsweise Korrosionsschutz, Entfettung sowie Adhäsionsprophylaxe.

Durch eine oszillierende oder rotierende Bewegung des Behälters entsteht eine Relativbewegung zwischen dem medizintechnischen Produkt und den Gleitschleifkörpern. Dadurch wird ein Materialabtrag am medizintechnischen Produkt, insbesondere an dessen Kanten, hervorgerufen. Das Oberflächenbild des medizintechnischen Produkts, die Rauheit, der Materialabtrag sowie die Entgratleistung lassen sich in vorteilhafter Weise gezielt durch zum Gleitschleifen verwendete Maschinen, Schleifkörper sowie durch optionale Zusatzmittel beeinflussen.

Die Gleitschleifkörper können ein Material aufweisen oder aus einem Material bestehen, welches ausgewählt ist aus der Gruppe bestehend aus Keramik, Kunststoff, Naturprodukt wie Walnussschalen, Stahl und Kombinationen davon.

Grundsätzlich können die Gleitschleifkörper regelmäßig und/oder unregelmäßig geformt vorliegen.

Die Gleitschleifkörper können insbesondere ecken- und/oder kantenfrei, wie beispielsweise ellipsoid-, toroid- oder kugelförmig, gestaltet sein.

Alternativ oder in Kombination können die Gleitschleifkörper Ecken und/oder Kanten aufweisen. Insbesondere können die Gleitschleifkörper polyederförmig, beispielsweise würfelförmig, quaderförmig, prismenförmig, pyramidenförmig oder spatförmig, gestaltet sein. Weiterhin können die Gleitschleifkörper insbesondere als gerade Prismen und/oder schiefe Prismen gestaltet sein.

Alternativ oder in Kombination können die Gleitschleifkörper kegel- und/oder kegelstumpfförmig gestaltet sein.

Ferner kann eine Mischung unterschiedlich gestalteter Gleitschleifkörper zum Gleitschleifen des medizintechnischen Produkts verwendet werden. Beispielsweise können ecken- und/oder kantenfreie Gleitschleifkörper und polyederförmige Gleitschleifkörper verwendet werden. Alternativ oder in Kombination können unterschiedlich gestaltete ecken- und/oder kantenfreie Gleitschleifkörper und/oder unterschiedliche polyederförmige Gleitschleifkörper verwendet werden. Bezüglich in Frage kommender Gestaltungen und Formen wird vollständig auf die in den vorherigen Absätzen beschriebenen Gestaltungen und Formen für die Gleitschleifkörper Bezug genommen.

Die Gleitschleifkörper können ferner wenigstens eine Abmessung, insbesondere wenigstens eine mittlere Abmessung, wie beispielsweise einen Durchmesser, insbesondere mittleren Durchmesser, und/oder eine Höhe, insbesondere mittlere Höhe, und/oder eine Länge, insbesondere mittlere Länge, im Bereich von 1 mm bis 80 mm aufweisen. Dabei soll unter dem Durchmesser von kugelförmig gestalteten Gleitschleifkörpern im Sinne der vorliegenden Erfindung der doppelte Radius eines einzelnen kugelförmig gestalteten Gleitschleifkörpers verstanden werden. Dagegen soll unter dem Durchmesser eines nicht kugelförmig gestalteten Gleitschleifkörpers im Sinne der vorliegenden Erfindung der größtmögliche Abstand zweier Punkte verstanden werden, welchen diese entlang einer Umfangslinie eines einzelnen, nicht kugelförmig gestalteten Gleitschleifkörpers zueinander einnehmen können. Die in diesem Absatz genannten mittleren Abmessungen können beispielsweise mittels Schüttdichte und/oder optischer Vermessung bestimmt werden. Das Gleitschleifen kann weiterhin als Trommelgleitschleifen, Vibrationsgleitschleifen, Tauchgleitspanen, Schleppschleifen, Fliehkraftgleitspanen oder Druckfließläppen durchgeführt werden.

Zum Bandschleifen des medizintechnischen Produkts werden vorzugsweise Schleifbänder verwendet. Hierfür können insbesondere Schleifbänder verwendet werden, welche über wenigstens zwei Rollen umlaufen. Die Schleifbänder weisen bevorzugt eine Körnung von 150 bis 1.200 auf. Die Zahl der Körnung orientiert sich dabei an der Maßeinheit Mesh, d.h. der Anzahl der Maschen eines Netzes pro Zoll (25,4 mm). Demzufolge passt beispielsweise ein Schleifmittel mit der Körnung 150 gerade noch durch ein Sieb mit 150 Maschen pro Zoll.

Erfindungsgemäß kann vor dem Durchführen von Schritt a) beispielsweise zunächst ein Gleitschleifen und anschließend ein Bandschleifen durchgeführt werden. Ein Bandschleifen kann vor allem im Hinblick auf das Behandeln eines sogenannten Abschattungsbereichs des medizintechnischen Produkts, aber auch außerhalb eines solchen Bereichs von Vorteil sein. Der Abschattungsbereich definiert den Bereich eines medizintechnischen Produkts, in welchem Gleitschleifkörper, insbesondere aufgrund der geometrischen Form und/oder Gestalt des medizintechnischen Produkts, nicht oder nur begrenzt auf der Oberfläche wirksam sind.

Alternativ kann die Oberfläche des medizintechnischen Produkts vor dem Durchführen von Schritt a) lediglich mittels Gleitschleifen geschliffen werden. Dadurch kann die Entstehung von auf Bandschleifen zurückgehende Kerben und/oder Anhebungen auf der Produktoberfläche vermieden und mithin die Korrosionsbeständigkeit des medizintechnischen Produkts zusätzlich verbessert werden.

Alternativ kann die Oberfläche des medizintechnischen Produkts vor dem Durchführen von Schritt a) lediglich mittels Bandschleifen geschliffen werden.

In weiterer Ausgestaltung der Erfindung wird beim oder zum Durchführen von Schritt a) die Oberfläche des medizintechnischen Produkts mit einem Strahlmittel, insbesondere duktilen, d.h. nicht spröden, Strahlmittel, behandelt. Die Verwendung eines duktilen Strahlmittels hat insbesondere den Vorteil, dass die Erzeugung von Kerben und/oder Mikrostrukturen, insbesondere in Form von Mikrospalten, verhindert oder wenigstens reduziert werden kann. Dadurch kann wiederum das Auftreten lokaler Spannungsspitzen im medizintechnischen Produkt vermieden oder wenigstens reduziert und insbesondere die Korrosionsbeständigkeit des medizintechnischen Produkts zusätzlich verbessert werden. Obendrein lässt sich durch die Verwendung eines derartigen Strahlmittels in vorteilhafter Weise die Kratzbeständigkeit des medizintechnischen Produkts (zusätzlich) verbessern.

Grundsätzlich kann das Strahlmittel ein Material aufweisen oder aus einem Material bestehen, welches ausgewählt ist aus der Gruppe bestehend aus Metall, Metalloxid, Legierung, Keramik, Kunststoff, pflanzlicher Stoff, Sand und Kombinationen davon.

Bei dem Metalloxid kann es sich insbesondere um Korund (Al₂O₃) handeln.

Bei dem Kunststoff kann es sich insbesondere um ein Harnstoff-, Phenol-, Polyester- oder Melamin-Harz handeln.

Bei der Keramik kann es sich insbesondere um Glas oder eine Mischkeramik handeln.

Bei der Legierung kann es sich um Stahl, insbesondere Edelstahl, handeln. Bevorzugt handelt es sich bei der Legierung um einen nichtrostenden Stahl, insbesondere nichtrostenden Edelstahl. Bezüglich geeigneter Edelstähle sei auf die noch folgende Beschreibung Bezug genommen.

Bei dem Sand kann es sich insbesondere um Granatsand handeln.

Bevorzugt weist das Strahlmittel ein Metall oder eine Legierung auf oder bevorzugt besteht das Strahlmittel aus einem Metall oder einer Legierung. Bezüglich geeigneter Metalle und Legierungen wird auf die vorherige Beschreibung Bezug genommen. Ein solches Strahlmittel hat insbesondere den Vorteil, dass es nicht bricht und mithin keine Einkerbung der Oberfläche des medizintechnischen Produkts verursacht. Obendrein kann ein Materialübertrag auf die Produktoberfläche reduziert oder sogar vollständig vermieden werden. Insgesamt kann dadurch die Korrosionsbeständigkeit des medizintechnischen Produkts zusätzlich verbessert sowie das Auftreten unerwünschter Eigenspannungen im Produkt vermieden werden. Außerdem ist ein solches Strahlmittel besonders geeignet, die Kratzbeständigkeit des medizintechnischen Produkts zu erhöhen.

Besonders bevorzugt weist das Strahlmittel Stahl, insbesondere Edelstahl, auf oder bevorzugt besteht das Strahlmittel aus Stahl, insbesondere Edelstahl. Durch ein solches Strahlmittel können die im letzten Absatz erwähnten Vorteile besonders stark zur Geltung gebracht werden.

Grundsätzlich kann das Strahlmittel regelmäßig und/oder unregelmäßig geformt, insbesondere als regelmäßig und/oder unregelmäßig geformte Strahlmittelkörper, vorliegen.

Ferner ist es besonders bevorzugt, dass das Strahlmittel ecken- und/oder kantenfrei, insbesondere als ecken- und/oder kantenfreie Strahlmittelkörper, gestaltet ist. Dadurch kann die Erzeugung von Einkerbungen auf der Oberfläche des medizintechnischen Produkts vermieden und mithin dessen Korrosionsbeständigkeit zusätzlich verbessert werden.

Grundsätzlich kann das Strahlmittel ellipsoid-, toroid-, kugel- oder perlenförmig gestaltet sein oder in Form entsprechend gestalteter Strahlmittelkörper vorliegen.

Vorzugsweise ist das Strahlmittel kugel- und/oder perlenförmig oder als kugel- und/oder perlenförmige Strahlmittelkörper gestaltet.

Alternativ oder in Kombination kann das Strahlmittel Ecken und/oder Kanten aufweisen. Insbesondere kann das Strahlmittel polyederförmig, beispielsweise würfelförmig, quaderförmig, prismenförmig, pyramidenförmig oder spatförmig, gestaltet sein oder als entsprechend gestaltete Strahlmittelkörper vorliegen. Das Strahlmittel kann ferner die Form eines geraden Prismas oder schiefen Prismas aufweisen oder in Form entsprechend gestalteter Strahlmittelkörper vorliegen.

Alternativ oder in Kombination kann das Strahlmittel kegel- und/oder kegelstumpfförmig gestaltet sein oder in Form von kegel- und/oder kegelstumpfförmigen Strahlmittelkörpern vorliegen.

Alternativ oder in Kombination kann das Strahlmittel in globulärer Form, beispielsweise in Form eines arrondierten Drahts, oder in Form entsprechend gestalteter Strahlmittelkörper vorliegen. Insbesondere bevorzugt kann das Strahlmittel in Form von Edelstahldrahtkörnern vorliegen.

Alternativ oder in Kombination kann das Strahlmittel in gebrochener Form, insbesondere in Form von gebrochenen Schleifmittelkörpern, vorliegen.

Ferner kann/können das Strahlmittel oder die Strahlmittelkörper wenigstens eine Abmessung, insbesondere wenigstens eine mittlere Abmessung, wie beispielsweise einen Durchmesser, insbesondere mittleren Durchmesser, und/oder eine Höhe, insbesondere mittlere Höhe, und/oder eine Länge, insbesondere mittlere Länge, im Bereich von 40 µm bis 2000 µm aufweisen. Dabei soll unter dem Durchmesser eines kugelförmig gestalteten Strahlmittels oder von kugelförmig gestalteten Strahlmittelkörpern im Sinne der vorliegenden Erfindung der doppelte Radius eines kugelförmig gestalteten Strahlmittels bzw. eines einzelnen kugelförmig gestalteten Strahlmittelkörpers verstanden werden. Dagegen soll unter dem Durchmesser eines nicht kugelförmig gestalteten Strahlmittels oder von nicht kugelförmig gestalteten Strahlmittelkörpern im Sinne der vorliegenden Erfindung der größtmögliche Abstand zweier Punkte verstanden werden, welchen diese entlang einer Umfangslinie eines nicht kugelförmig gestalteten Strahlmittels bzw. eines einzelnen nicht kugelförmig gestalteten Strahlmittelkörpers zueinander einnehmen können. Die in diesem Absatz genannten mittleren Abmessungen können beispielsweise mittels Laserbeugung oder Siebanalyse bestimmt werden.

Zur Beschleunigung des Strahlmittels oder der Strahlmittelkörper auf die Oberfläche des medizintechnischen Produkts können beispielsweise Druckstrahlanlagen, Injektorstrahlanlagen oder Schleuderradanlagen eingesetzt werden. Kommt eine Druckstrahl- oder Injektorstrahlanlage zum Einsatz, können Drücke von 1 bar bis 6 bar verwendet werden.

Üblicherweise wird die Oberfläche des medizintechnischen Produkts beim oder zum Durchführen von Schritt b) in einer Elektrolytlösung anodisch abgetragen, d.h. das medizintechnische Produkt bildet beim oder zum Durchführen von Schritt b) üblicherweise die Anode in einer elektrochemischen Zelle.

In weiterer Ausgestaltung der Erfindung wird der Schritt b) während eines Zeitraumes von 30 s bis 130 s, insbesondere 60 s bis 100 s, vorzugsweise 90 s, durchgeführt.

Die Schritte b) und d) werden jeweils bei gleicher Spannung, insbesondere Gleichspannung, durchgeführt.

Der Schritt b) und der Schritt d) werden jeweils bei/mit einer Spannung, insbesondere Gleichspannung, von 2,0 V bis 10,0 V, bevorzugt 5 V bis 9 V, besonders bevorzugt 7 V bis 8 V, durchgeführt. Durch die in diesem Absatz offenbarten Spannungswerte sind die Ausbildung eines gleichmäßigen Polierfilms sowie ein gleichmäßiger Übertrag des Metalls oder der Legierung in die Elektrolytlösung erzielbar.

Ferner können/kann der Schritt b) und/oder der Schritt d) bei/mit einer konstanten oder variierenden Spannung, insbesondere Gleichspannung, durchgeführt werden. Bezüglich geeigneter Spannungsbereiche/-werte wird auf die im vorangegangenen Absatz offenbarten Spannungen Bezug genommen.

Alternativ oder in Kombination wird der Schritt b) und der Schritt d) jeweils bei/mit einer Stromdichte von 5 A/dm² bis 50 A/dm², insbesondere 8 A/dm² bis 40 A/dm², bevorzugt 15 A/dm² bis 20 A/dm², durchgeführt.

Ferner können/kann der Schritt b) und/oder der Schritt d) mehrmals, insbesondere zwei-, dreioder viermal, durchgeführt werden. Insbesondere können die Schritte b) und d) unterschiedlich oft wiederholt werden. Durch eine mehrmalige Wiederholung der Schritte b) und d) lassen sich mit besonderem Vorteil geometrische Besonderheiten des medizintechnischen Produkts, wie zum Beispiel ein Schluss des medizintechnischen Produkts, gleichmäßig bearbeiten, ohne dass eine relevante Abschattung entsteht. Der Schluss des medizintechnischen Produkts kann zum Beispiel in zwei Positionen bearbeitet werden, damit nur geringe Abschattungen entstehen. Alternativ kann es bevorzugt sein, dass das medizintechnische Produkt während des Durchführens von Schritt b) und/oder d) langsam artikuliert wird.

Alternativ können/kann der Schritt b) und/oder der Schritt d) nur einmal durchgeführt werden.

Ferner kann das medizintechnische Produkt vor dem Durchführen von Schritt b) und/oder vor dem Durchführen von Schritt d) gereinigt und/oder entfettet werden.

Üblicherweise wird die Oberfläche des medizintechnischen Produkts beim oder zum Durchführen von Schritt c) (ebenfalls) in einer Elektrolytlösung anodisch abgetragen, d.h. das medizintechnische Produkt bildet beim oder zum Durchführen von Schritt c) üblicherweise (ebenfalls) die Anode in einer elektrochemischen Zelle.

In weiterer Ausgestaltung der Erfindung wird der Schritt c) während eines Zeitraumes von 30 s bis 130 s, insbesondere 60 s bis 100 s oder 70 s bis 110 s, vorzugsweise 90 s oder 100 s, durchgeführt. Grundsätzlich kann der Schritt c) während eines kürzeren oder längeren Zeitraumes durchgeführt werden als der Schritt b). Bevorzugt werden die Schritte b) und c) während eines gleich langen Zeitraumes durchgeführt.

Weiter wird der Schritt c) bei/mit einer, vorzugsweise an der Anode (an dem zu oberflächenbehandelnden oder -bearbeitenden und/oder herzustellenden medizintechnischen Produkt) gemessenen, Spannung, insbesondere Gleichspannung, von ≤ 2,1 V, insbesondere < 2 V, insbesondere 1,2 V bis 1,8 V, bevorzugt 1,4 V bis 1,7 V, besonders bevorzugt 1,4 V bis 1,5 V oder 1,45 V bis 1,65 V, durchgeführt. Dadurch kommen die durch den elektrochemischen Ätzschritt erzielbaren erfindungsgemäßen Vorteile besonders stark zur Geltung. Die Messung der Spannung an der Anode (an dem zu oberflächenbehandelnden oder -bearbeitenden und/oder herzustellenden medizintechnischen Produkt) erfolgt vorzugsweise über eine Silber-Silberchlorid-Elektrode. Die ermittelten Spannungen werden anschließend auf eine Standardwasserstoffelektrode umgerechnet. Üblicherweise wird die Spannung an der Stromquelle eingestellt, ohne zu wissen, welcher Teil der Spannung an der Anode anliegt und wie viel an restlichen Widerständen (zum Beispiel Leitungen, Elektrolyt, usw.) anliegt. Bei der vorliegenden Erfindung ist vorzugsweise die genaue Spannung an der Anode entscheidend.

Ferner kann der Schritt c) bei/mit einer konstanten oder variierenden Spannung, insbesondere Gleichspannung, durchgeführt werden. Bezüglich geeigneter Spannungsbereiche/-werte wird auf die im vorangegangenen Absatz offenbarten Spannungen Bezug genommen.

Alternativ oder in Kombination wird der Schritt c) bei/mit einer Stromdichte von 1,4 A/dm² bis 2,4 A/dm², insbesondere 1,6 A/dm² bis 2,2 A/dm², vorzugweise 1,8 A/dm² bis 2,0 A/dm², durchgeführt. Durch die in diesem Absatz offenbarten (niedrigen) Stromdichten lässt sich ein elektrochemisches Ätzen der Oberfläche des medizintechnischen Produkts in zeitlicher Hinsicht besonders gut steuern.

Ferner kann der Schritt c) bei einer Temperatur von 20 °C bis 90 °C, insbesondere 50 °C bis 80 °C, vorzugweise 70 °C bis 80 °C, durchgeführt werden.

Bevorzugt wird der Schritt c) mehrmals, insbesondere zwei-, drei- oder viermal, durchgeführt.

Durch eine mehrmalige Wiederholung von Schritt c) lassen sich (ebenfalls) mit besonderem Vorteil geometrische Besonderheiten des medizintechnischen Produkts, wie zum Beispiel ein Schluss des medizintechnischen Produkts, gleichmäßig bearbeiten, ohne dass relevante Abschattungen entstehen. Der Schluss des medizintechnischen Produkts kann hierzu zum Beispiel (ebenfalls) in zwei Positionen bearbeitet werden, damit nur geringe Abschattungen entstehen. Alternativ kann es bevorzugt sein, dass das medizintechnische Produkt während des Durchführens von Schritt c) (ebenfalls) langsam artikuliert wird.

Alternativ kann der Schritt c) nur einmal durchgeführt werden.

Üblicherweise wird die Oberfläche des medizintechnischen Produkts beim oder zum Durchführen von Schritt d) (ebenfalls) in einer Elektrolytlösung anodisch abgetragen, d.h. das medizintechnische Produkt bildet beim oder zum Durchführen von Schritt d) üblicherweise (ebenfalls) die Anode in einer elektrochemischen Zelle.

In weiterer Ausgestaltung der Erfindung wird der Schritt d) während eines kürzeren Zeitraumes durchgeführt als der Schritt b). Bevorzugt wird der Schritt d) während eines Zeitraumes von 5 s bis 20 s, insbesondere 7 s bis 15 s, vorzugsweise 10 s, durchgeführt.

In weiterer Ausgestaltung der Erfindung wird zum Durchführen der Schritte b), c) und d) jeweils eine saure, wässrige Elektrolytlösung, insbesondere aufweisend eine Mineralsäure oder ein Gemisch von Mineralsäuren, verwendet. Bevorzugt wird die Mineralsäure ausgewählt aus der Gruppe bestehend aus Phosphorsäure, Schwefelsäure und ein Gemisch davon. Eine phosphorsäure- und/oder schwefelsäurehaltige, wässrige Elektrolytlösung hat sich dabei als besonders vorteilhaft für das Behandeln der Oberfläche eines medizintechnischen Produkts aus Edelstahl, insbesondere nichtrostendem oder korrosionsbeständigem Edelstahl, herausgestellt.

Die saure, wässrige Elektrolytlösung kann einen Mineralsäureanteil von 50 Gew.-% bis 95 Gew.%, insbesondere 60 Gew.-% bis 95 Gew.-%, vorzugsweise 75 Gew.-% bis 95 Gew.-%, aufweisen, bezogen auf das Gesamtgewicht der sauren, wässrigen Elektrolytlösung. Insbesondere kann die saure, wässrige Elektrolytlösung einen Phosphorsäureanteil von 20 Gew.-% bis 70 Gew.-%, insbesondere 30 Gew.-% bis 60 Gew.-%, vorzugsweise 40 Gew.-% bis 50 Gew.-%, und/oder einen Schwefelsäureanteil von 10 Gew.-% bis 70 Gew.-%, insbesondere 20 Gew.-% bis 60 Gew.%, vorzugsweise 30 Gew.-% bis 50 Gew.-%, aufweisen, jeweils bezogen auf das Gesamtgewicht der sauren, wässrigen Elektrolytlösung.

Bei der sauren, wässrigen Elektrolytlösung kann es sich ferner um eine gealterte, saure, wässrige Elektrolytlösung handeln.

Die saure, wässrige Elektrolytlösung kann weiter Zusatzstoffe, wie beispielsweise oberflächenaktive Substanzen, aufweisen.

Vorteilhaft ist ferner, dass die Aggressivität der sauren, wässrigen Elektrolytlösung gezielt über deren Wasseranteil gesteuert werden kann. Beispielsweise kann die saure, wässrige Elektrolytlösung einen Wasseranteil von 5 Gew.-% bis 25 Gew.-%, insbesondere 5 Gew.-% bis 15 Gew.-%, vorzugsweise 5 Gew.-% bis 10 Gew.-%, aufweisen, bezogen auf das Gesamtgewicht der sauren, wässrigen Elektrolytlösung.

Grundsätzlich können die Schritte b), c) und d) jeweils mit einer unterschiedlichen sauren, wässrigen Elektrolytlösung, insbesondere aufweisend eine Mineralsäure oder ein Gemisch von Mineralsäuren, durchgeführt werden. Alternativ können die Schritte b) und d) jeweils mit der gleichen sauren, wässrigen Elektrolytlösung, insbesondere aufweisend eine Mineralsäure oder ein Gemisch von Mineralsäuren, durchgeführt werden. Insbesondere kann sich die saure, wässrige Elektrolytlösung, insbesondere aufweisend eine Mineralsäure oder ein Gemisch von Mineralsäuren, zum Durchführen der Schritte b) und d) von der sauren, wässrigen Elektrolytlösung, insbesondere aufweisend eine Mineralsäure oder ein Gemisch von Mineralsäuren, zum Durchführen von Schritt c) unterscheiden, insbesondere in Bezug auf die Mineralsäure und/oder den Mineralsäureanteil. Bezüglich geeigneter Mineralsäuren sowie Mineralsäureanteile wird vollumfänglich auf die vorangegangenen Ausführungen Bezug genommen.

In weiterer Ausgestaltung der Erfindung wird nach dem Durchführen von Schritt d) ein Schritt e) Behandeln der mattierten, elektropolierten, elektrochemisch geätzten und nochmals elektropolierten Oberfläche des medizintechnischen Produkts mit einer Passiviersäure oder einer passiviersäurehaltigen Lösung durchgeführt. In anderen Worten wird in weiterer Ausgestaltung der Erfindung nach dem Durchführen von Schritt d) ein Schritt e) Passivieren der mattierten, elektropolierten, elektrochemisch geätzten und nochmals elektropolierten Oberfläche des medizintechnischen Produkts durchgeführt. Hierdurch kann mit besonderem Vorteil die Ausbildung einer Passivschicht auf der Oberfläche des medizintechnischen Produkts zusätzlich verstärkt oder begünstigt und mithin die Korrosionsbeständigkeit des medizintechnischen Produkts zusätzlich verbessert werden. Im Fall eines medizintechnischen Produkts aus einem chromhaltigen oder chromlegierten Edelstahl lassen sich durch einen Passivierungsschritt beispielsweise verstärkte Chromoxidschichten auf der Oberfläche des medizintechnischen Produkts ausbilden.

In weiterer Ausgestaltung der Erfindung wird als Passiviersäure Zitronensäure, Salpetersäure oder ein Gemisch davon verwendet.

Als passiviersäurehaltige Lösung zum Durchführen von Schritt e) wird bevorzugt eine wässrige, zitronensäurehaltige Lösung, insbesondere mit einem Zitronensäureanteil von 5 Gew.-% bis 60 Gew.-%, bezogen auf das Gesamtgewicht der wässrigen, zitronensäurehaltigen Lösung, verwendet. Alternativ kann als passiviersäurehaltige Lösung zum Durchführen von Schritt e) bevorzugt eine wässrige, salpetersäurehaltige Lösung, insbesondere mit einem Salpetersäureanteil von 5 Gew.-% bis 60 Gew.-%, bezogen auf das Gesamtgewicht der wässrigen, salpetersäurehaltigen Lösung, verwendet werden.

Die Verwendung von Zitronensäure hat sowohl unter Gesundheits- als auch Arbeitssicherheitsgesichtspunkten Vorteile gegenüber einer Verwendung von Salpetersäure. Obendrein lassen sich mittels Zitronensäure im Fall von medizintechnischen Produkten aus chromhaltigem oder chromlegiertem Edelstahl dickere Chromoxidschichten realisieren, als dies bei Verwendung von Salpetersäure der Fall ist, da Letztere im Fall eines solchen Edelstahls auch den Anteil anderer Legierungsbestandteile reduziert.

Zum Durchführen von Schritt e) kann das medizintechnische Produkt beispielsweise in die Passiviersäure oder passiviersäurehaltige Lösung eingetaucht werden. Alternativ kann die Passiviersäure oder die passiviersäurehaltige Lösung auf die Oberfläche des medizintechnischen Produkts gesprüht oder gegossen werden.

Ferner kann der Schritt e) während eines Zeitraumes von 2 min bis 2 h, insbesondere 5 min bis 60 min, bevorzugt 10 min bis 30 min, durchgeführt werden.

Ferner kann der Schritt e) in einem Temperaturbereich von 20 °C bis 80 °C, insbesondere 30 °C bis 65 °C, bevorzugt 50 °C bis 60 °C, durchgeführt werden.

Ferner kann zwischen dem Schritt d) und dem Schritt e) ein Schritt de) Reinigen und/oder Entfetten des medizintechnischen Produkts, insbesondere ein Reinigen und/oder Entfetten der mattierten, elektropolierten, elektrochemisch geätzten und nochmals elektropolierten Oberfläche des medizintechnischen Produkts, durchgeführt werden.

In weiterer Ausgestaltung der Erfindung wird nach dem Durchführen von Schritt e) ein Schritt f) Verpacken und/oder Kennzeichnen, insbesondere Etikettieren, des medizintechnischen Produkts durchgeführt. Bevorzugt wird zwischen dem Schritt e) und dem Schritt f) ein Schritt ef) Sterilisieren, insbesondere Dampfsterilisieren, des medizintechnischen Produkts durchgeführt. Alternativ kann es bevorzugt sein, dass nach dem Durchführen von Schritt f) ein Schritt g) Sterilisieren, insbesondere Dampfsterilisieren, des medizintechnischen Produkts durchgeführt wird.

In weiterer Ausgestaltung der Erfindung weist das medizintechnische Produkt Stahl, bevorzugt Edelstahl, auf oder besteht das medizintechnische Produkt aus Stahl, bevorzugt Edelstahl.

Unter dem Ausdruck "Edelstahl" soll im Sinne der vorliegenden Erfindung (in Übereinstimmung nach EN 10020) ein legierter oder unlegierter Stahl mit besonderem Reinheitsgrad, beispielsweise mit einem Schwefel- und/oder Phosphormassenanteil ≤ 0,025 %, insbesondere < 0,025 %, verstanden werden.

Der Edelstahl kann insbesondere wenigstens ein Legierungselement, ausgewählt aus der Gruppe bestehend aus Chrom, Nickel, Molybdän, Titan, Niob, Wolfram, Vanadium, Kobalt und Kombinationen davon, aufweisen.

Insbesondere kann der Edelstahl einen Chrommassenanteil von 10 % bis 25 % aufweisen.

Weiter bevorzugt handelt es sich bei dem Edelstahl um einen nichtrostenden oder korrosionsbeständigen Edelstahl.

Weiter bevorzugt handelt es sich bei dem Edelstahl um einen chromhaltigen oder chromlegierten Edelstahl. Vorzugsweise handelt es sich bei dem Edelstahl um einen chromhaltigen, korrosionsbeständigen Edelstahl oder um einen chromlegierten, korrosionsbeständigen Edelstahl.

Ferner kann es sich bei dem Edelstahl insbesondere um einen martensitischen, ferritischen oder austenitischen Edelstahl handeln.

Vorzugsweise handelt es sich bei dem Edelstahl um einen martensitischen, korrosionsbeständigen Edelstahl, insbesondere um einen sogenannten Kohlenstoffmartensit, d.h. einen korrosionsbeständigen Edelstahl mit Chrom und Kohlenstoff als Hauptlegierungsbestandteile, oder um einen sogenannten Nickelmartensit, d.h. um einen korrosionsbeständigen Edelstahl mit Nickel als Hauptlegierungsbestandteil, gemäß ISO 7153-1.

Insbesondere kann es sich bei dem Edelstahl um einen martensitischen Edelstahl mit einem Chrommassenanteil von 10,5 % bis 13 % und/oder einem Kohlenstoffmassenanteil von 0,2 % bis 1 % handeln.

Alternativ kann es sich bei dem Edelstahl insbesondere um einen austenitischen Edelstahl mit einem Chrommassenanteil von 16 % bis 21 % und/oder einem Kohlenstoffmassenanteil von 0,02 % bis 0,12 % handeln.

Alternativ kann es sich bei dem Edelstahl insbesondere um einen ferritischen Edelstahl mit einem Chrommassenanteil von 12 % bis 18 % und/oder einem Kohlenstoffmassenanteil von < 0,2 % handeln.

Beispielsweise kann es sich bei dem Edelstahl um einen Edelstahl mit der Werkstoffkurzbezeichnung X12Cr13 (Werkstoffnummer 1.4006) handeln. Hierbei handelt es sich um einen martensitischen Edelstahl mit einem Kohlenstoffmassenanteil von 0,08 % bis 0,15 %, einem Chrommassenanteil von 11,5 % bis 13,5 % sowie einem Nickelmasseanteil von ≤ 0,75 %.

Alternativ kann es sich bei dem Edelstahl um einen martensitischen, korrosionsbeständigen Edelstahl mit der Werkstoffkurzbezeichnung X12CrS13 (Werkstoffnummer 1.4005) handeln. Dieser Edelstahl weist einen Kohlenstoffmassenanteil von 0,08 % bis 0,15 %, einen Chrommassenanteil von 12,0 % bis 14,0 % sowie eine Molybdänmassenanteil ≤ 0,60 % sowie optional einen Schwefelmassenanteil von 0,15 % bis 0,35 % auf.

Alternativ kann es sich bei dem Edelstahl um einen martensitischen, korrosionsbeständigen Edelstahl mit der Werkstoffkurzbezeichnung X20Cr13 (Werkstoffnummer: 1.4021) handeln. Dieser Edelstahl weist einen Kohlenstoffmassenanteil von 0,16 % bis 0,25 % und einen Chrommassenanteil von 12,0 % bis 14,0 % auf.

Alternativ kann es sich bei dem Edelstahl um einen martensitischen, korrosionsbeständigen Edelstahl mit der Werkstoffkurzbezeichnung X15Cr13 (Werkstoffnummer: 1.4024) handeln. Dieser Edelstahl weist einen Kohlenstoffmassenanteil von 0,12 % bis 0,17 % und einen Chrommassenanteil von 12,0 % bis 14,0 % auf.

Alternativ kann es sich bei dem Edelstahl um einen martensitischen, korrosionsbeständigen Edelstahl mit der Werkstoffkurzbezeichnung X30Cr13 (Werkstoffnummer: 1.4028) handeln. Dieser Edelstahl weist einen Kohlenstoffmassenanteil von 0,26 % bis 0,35 % sowie einen Chrommassenanteil von 12,0 % bis 14,0 % auf.

Alternativ kann es sich bei dem Edelstahl um einen martensitischen, korrosionsbeständigen Edelstahl mit der Werkstoffkurzbezeichnung X46Cr13 (Werkstoffnummer: 1.4034) handeln. Dieser Edelstahl weist einen Kohlenstoffmassenanteil von 0,43 % bis 0,50 % sowie einen Chrommassenanteil von 12,5 % bis 14,5 % auf.

Alternativ kann es sich bei dem Edelstahl um einen martensitischen, korrosionsbeständigen Edelstahl mit der Werkstoffkurzbezeichnung X50CrMoV15 (Werkstoffnummer: 1.4116) handeln.

Dieser Edelstahl weist einen Kohlenstoffmassenanteil von 0,45 % bis 0,55 %, einen Chrommassenanteil von 14,0 % bis 15,0 %, einen Molybdänmassenanteil von 0,50 % bis 0,80 % sowie einen Vanadiummassenanteil von 0,10 % bis 0,20 % auf.

Alternativ kann es sich bei dem Edelstahl um einen martensitischen, korrosionsbeständigen Edelstahl mit der Werkstoffkurzbezeichnung X17CrNi16-2 (Werkstoffnummer: 1.4057) handeln. Dieser Edelstahl weist einen Kohlenstoffmassenanteil von 0,12 % bis 0,22 %, einen Chrommassenanteil von 15,0 % bis 17,0 % sowie einen Nickelmassenanteil von 1,5 % bis 2,5 % auf.

Alternativ kann es sich bei dem Edelstahl um einen martensitischen, korrosionsbeständigen Edelstahl mit der Werkstoffkurzbezeichnung X39CrMo17-1 (Werkstoffnummer: 1.4122) handeln. Dieser Edelstahl weist einen Kohlenstoffmassenanteil von 0,33 % bis 0,45 %, einen Chrommassenanteil von 15,5 % bis 17,5 %, einen Molybdänmassenanteil von 0,8 % bis 1,3 % sowie einen Nickelmassenanteil ≤ 1,0 % auf.

Alternativ kann es sich bei dem Edelstahl um einen martensitischen, korrosionsbeständigen Edelstahl mit der Werkstoffkurzbezeichnung X14CrMoS17 (Werkstoffnummer: 1.4104) handeln. Dieser Edelstahl weist einen Kohlenstoffmassenanteil von 0,10 % bis 0,17 %, einen Chrommassenanteil von 15,5 % bis 17,5 %, einen Molybdänmassenanteil von 0,20 % bis 0,60 % sowie einen Schwefelmassenanteil von 0,15 % bis 0,35 % auf.

Alternativ kann es sich bei dem Edelstahl um einen martensitischen, korrosionsbeständigen Edelstahl mit der Werkstoffkurzbezeichnung X3CrNiMo13-4 (Werkstoffnummer: 1.4313) handeln. Dieser Edelstahl weist einen Kohlenstoffmassenanteil ≤ 0,05 %, einen Chrommassenanteil von 12,0 % bis 14,0 %, einen Molybdänmassenanteil von 0,3 % bis 0,7 % sowie einen Nickelmassenanteil von 3,5 % bis 4,5 % auf.

Alternativ kann es sich bei dem Edelstahl um einen martensitischen, korrosionsbeständigen Edelstahl mit der Werkstoffkurzbezeichnung X4CrNiMo16-5-1 (Werkstoffnummer: 1.4418) handeln. Dieser Edelstahl weist einen Kohlenstoffmassenanteil ≤ 0,06 %, einen Chrommassenanteil von 15,0 % bis 17,0 %, einen Molybdänmassenanteil von 0,80 % bis 1,50 % sowie einen Nickelmassenanteil von 4,0 % bis 6,0 % auf.

Alternativ kann es sich bei dem Edelstahl um einen martensitischen Edelstahl mit der Werkstoffkurzbezeichnung X65Cr13 handeln. Dieser Edelstahl weist einen Kohlenstoffmassenanteil von 0,58 % bis 0,70 %, einen Chrommassenanteil von 12,5 % bis 14,5 %, einen Manganmassenanteil ≤ 1,00 %, einen Siliziummassenanteil ≤ 1,00 %, einen Phosphormassenanteil von 0,04 % sowie einen Schwefelmassenanteil von 0,015 % auf.

Alternativ kann es sich bei dem Edelstahl um einen martensitischen Edelstahl mit der Werkstoffkurzbezeichnung X30CrMoN15-1 (Werkstoffnummer: 1.4108) handeln. Dieser Edelstahl weist einen Kohlenstoffmassenanteil von 0,25 % bis 0,35 %, einen Chrommassenanteil von 14,0 % bis 16,0 %, einen Molybdänmassenanteil von 0,85 % bis 1,10 %, einen Nickelmassenanteil von 0,50 %, einen Manganmassenanteil von 1,00 %, einen Siliziummassenanteil von 1,00 % sowie einen Stickstoffmassenanteil von 0,03 % bis 0,50% auf.

Alternativ kann es sich bei dem Edelstahl um einen martensitischen Edelstahl mit der Werkstoffkurzbezeichnung X70CrMo15 (Werkstoffnummer: 1.4109) handeln. Dieser Edelstahl weist einen Kohlenstoffmassenanteil von 0,60 % bis 0,75 %, einen Chrommassenanteil von 14,0 % bis 16,0 %, einen Molybdänmassenanteil von 0,40 % bis 0,80 %, einen Manganmassenanteil ≤ 1,00 %, einen Siliziummassenanteil ≤ 0,70 %, einen Phosphormassenanteil von 0,04 % sowie einen Schwefelmassenanteil von 0,015 % auf.

Alternativ kann es sich bei dem Edelstahl um einen martensitischen Edelstahl mit der Werkstoffkurzbezeichnung X90CrMoV18 (Werkstoffnummer: 1.4112) handeln. Dieser Edelstahlweist einen Kohlenstoffmassenanteil von 0,90 %, einen Chrommassenanteil von 17 % bis 19 % sowie einen Molybdänmassenanteil von 0,90 % auf.

Alternativ kann es sich bei dem Edelstahl um einen martensitischen Edelstahl mit der Werkstoffkurzbezeichnung X38CrMoV15 (Werkstoffnummer: 1.4117) handeln. Dieser Edelstahl weist einen Kohlenstoffmassenanteil von 0,38 %, einen Chrommassenanteil von 14 % bis 15 % sowie einen Molybdänmassenanteil von 0,50 % auf.

Alternativ kann es sich bei dem Edelstahl um einen martensitischen Edelstahl mit der Werkstoffkurzbezeichnung X150CrMo17 (Werkstoffnummer: 1.4125) handeln. Dieser Edelstahl weist einen Kohlenstoffmassenanteil von 1,10 %, einen Chrommassenanteil von 17 % sowie einen Molybdänmassenanteil von 0,60 % auf.

Alternativ kann es sich bei dem Edelstahl um einen martensitischen Edelstahl mit der Werkstoffkurzbezeichnung X22CrMoNiS13-1 (Werkstoffnummer: 1.4121) handeln. Dieser Edelstahl weist einen Kohlenstoffmassenanteil von 0,20 % bis 0,25 %, einen Chrommassenanteil von 12,0 % bis 14,0 %, einen Molybdänmassenanteil von 1,00 % bis 1,50 %, einen Nickelmassenanteil von 0,80 % bis 1,20 %, einen Manganmassenanteil von 1,00 % bis 1,50 %, einen Siliziummassenanteil ≤ 1,00 %, einen Phosphormassenanteil von 0,045 % sowie einen Schwefelmassenanteil von 0,15 % bis 0,25 % auf.

Alternativ kann es sich bei dem Edelstahl um einen martensitischen Edelstahl mit der Werkstoffkurzbezeichnung X40CrMoVN16-2 (Werkstoffnummer: 1.4123) handeln. Dieser Edelstahlweist einen Kohlenstoffmassenanteil von 0,35 % bis 0,50 %, einen Chrommassenanteil von 14,0 % bis 16,0 %, einen Molybdänmassenanteil von 1,00 % bis 2,50 %, einen Nickelmassenanteil von 0,5 %, einen Manganmassenanteil ≤ 1,00 %, einen Siliziummassenanteil ≤ 1,00 %, einen Phosphormassenanteil von 0,04 % sowie einen Schwefelmassenanteil von 0,015 % auf.

Alternativ kann es sich bei dem Edelstahl um einen martensitischen Edelstahl mit der Werkstoffkurzbezeichnung X105CrMo17 (Werkstoffnummer: 1.4125) handeln. Dieser Edelstahl weist einen Kohlenstoffmassenanteil von 0,95 % bis 1,20 %, einen Chrommassenanteil von 16,0 % bis 18,0 %, einen Molybdänmassenanteil von 0,04 % bis 0,80 %, einen Manganmassenanteil von maximal 1,00 %, einen Silziummassenanteil von maximal 1,00 %, einen Phosphormassenanteil von maximal 0,040 % sowie einen Schwefelmassenanteil von maximal 0,015 % auf.

Alternativ kann es sich bei dem Edelstahl um einen ausscheidungshärtenden, korrosionsbeständigen Edelstahl mit der Werkstoffkurzbezeichnung X5CrNiCuNb16-4 (Werkstoffnummer: 1.4542) handeln. Dieser Edelstahl weist einen Kohlenstoffmassenanteil ≤ 0,07 %, einen Chrommassenanteil von 15,0 % bis 17,0 %, einen Molybdänmassenanteil ≤ 0,60 %, einen Nickelmassenanteil von 3,0 % bis 5,0 %, einen Kupfermassenanteil von 3,0 % bis 5,0 % sowie einen Niobmassenanteil von maximal 0,45 % auf.

Alternativ kann es sich bei dem Edelstahl um einen ausscheidungshärtenden, korrosionsbeständigen Edelstahl mit der Werkstoffkurzbezeichnung X7CrNiAl17-7 (Werkstoffnummer: 1.4568) handeln. Dieser Edelstahl weist einen Kohlenstoffmassenanteil ≤ 0,09 %, einen Chrommassenanteil von 16,0 % bis 18,0 %, einen Nickelmassenanteil von 6,5 % bis 7,8 % sowie einen Aluminiummassenanteil von 0,70 % bis 1,50 % auf.

Alternativ kann es sich bei dem Edelstahl um einen ausscheidungshärtenden, korrosionsbeständigen Edelstahl mit der Werkstoffkurzbezeichnung X5CrNiMoCuNb14-5 (Werkstoffnummer: 1.4594) handeln. Dieser Edelstahl weist einen Kohlenstoffmassenanteil ≤ 0,07 %, einen Chrommassenanteil von 13,0 % bis 15,0 %, einen Molybdänmassenanteil von 1,20 % bis 2,00 %, einen Nickelmassenanteil von 5,0 % bis 6,0 %, einen Kupfermassenanteil von 1,20 % bis 2,00 % sowie einen Niobmassenanteil von 0,15 % bis 0,60 % auf.

Alternativ kann es sich bei dem Edelstahl um einen ausscheidungshärtenden, korrosionsbeständigen Edelstahl mit der Werkstoffkurzbezeichnung X3CrNiTiMb12-9 (Werkstoffnummer: 1.4543) handeln. Dieser Edelstahl weist einen Kohlenstoffmassenanteil ≤ 0,03 %, einen Chrommassenanteil von 11,0 % bis 12,5 %, einen Molybdänmassenanteil ≤ 0,50%, einen Nickelmassenanteil von 3,00 % bis 5,00 %, einen Titanmassenanteil von ≤ 0,90 % bis 1,40 %, einem Kupfermassenanteil von 1,50 % bis 2,50 %, einen Niobmassenanteil von 0,10 % bis 0,50 %, einen Manganmassenanteil von 0,50 %, einen Siliziummassenanteil von 0,50 %, einen Phosphormassenanteil ≤ 0,02% sowie einen Schwefelmassenanteil ≤ 0,015 %.

Alternativ kann es sich bei dem Edelstahl um einen ferritischen, korrosionsbeständigen Edelstahl mit der Werkstoffkurzbezeichnung X2CrNi12 (Werkstoffnummer: 1.4003) handeln. Dieser Edelstahl weist einen Kohlenstoffmassenanteil ≤ 0,03 %, einen Chrommassenanteil von 10,5 % bis 12,5 %, einen Nickelmassenanteil von 0,3 % bis 1,00 % sowie einen Stickstoffanteil ≤ 0,03 % auf.

Alternativ kann es sich bei dem Edelstahl um einen ferritischen, korrosionsbeständigen Edelstahl mit der Werkstoffkurzbezeichnung X2CrNi12 (Werkstoffnummer: 1.4512) handeln. Dieser Edelstahl weist einen Kohlenstoffmassenanteil ≤ 0,03 %, einen Chrommassenanteil von 10,5 % bis 12,5 % sowie einen Titanmassenanteil von maximal 0,65 % auf.

Alternativ kann es sich bei dem Edelstahl um einen ferritischen, korrosionsbeständigen Edelstahl mit der Werkstoffkurzbezeichnung X6Cr17 (Werkstoffnummer: 1.4016) handeln. Dieser Edelstahl weist einen Kohlenstoffmassenanteil ≤ 0,08 % sowie einen Chrommassenanteil von 16,0 % bis 18,0 % auf.

Alternativ kann es sich bei dem Edelstahl um einen ferritischen, korrosionsbeständigen Edelstahl mit der Werkstoffkurzbezeichnung X3CrTi17 (Werkstoffnummer: 1.4510) handeln. Dieser Edelstahl weist einen Kohlenstoffmassenanteil ≤ 0,05 %, einen Chrommassenanteil von 16,0 % bis 18,0 % sowie einen Titanmassenanteil von maximal 0,80 % auf.

Alternativ kann es sich bei dem Edelstahl um einen ferritischen, korrosionsbeständigen Edelstahl mit der Werkstoffkurzbezeichnung X6CrMoS17 (Werkstoffnummer: 1.4105) handeln. Dieser Edelstahl weist einen Kohlenstoffmassenanteil ≤ 0,08 %, einen Chrommassenanteil von 16,0 % bis 18,0 %, einen Molybdänmassenanteil von 0,20 % bis 0,60 % sowie einen Schwefelmassenanteil von 0,15 % bis 0,35 % auf.

Alternativ kann es sich bei dem Edelstahl um einen ferritischen, korrosionsbeständigen Edelstahl mit der Werkstoffkurzbezeichnung X3CrNb17 (Werkstoffnummer: 1.4511) handeln. Dieser Edelstahl weist einen Kohlenstoffmassenanteil ≤ 0,05 %, einen Chrommassenanteil von 16,0 % bis 18,0 % sowie einen Niobmassenanteil von maximal 1,00 % auf.

Alternativ kann es sich bei dem Edelstahl um einen ferritischen, korrosionsbeständigen Edelstahl mit der Werkstoffkurzbezeichnung X2CrTiNb18 (Werkstoffnummer: 1.4509) handeln. Dieser Edelstahl weist einen Kohlenstoffmassenanteil ≤ 0,03 %, einen Chrommassenanteil von 17,5 % bis 18,5 %, einen Niobmassenanteil von maximal 1,00 % sowie einen Titanmassenanteil von 0,10 % bis 0,60 % auf.

Alternativ kann es sich bei dem Edelstahl um einen ferritischen, korrosionsbeständigen Edelstahl mit der Werkstoffkurzbezeichnung X6CrMo17-1 (Werkstoffnummer: 1.4113) handeln. Dieser Stahl weist einen Kohlenstoffmassenanteil ≤ 0,08 %, einen Chrommassenanteil von 16,0 % bis 18,0 % sowie einen Molybdänmassenanteil von 0,90 % bis 1,40 % auf.

Alternativ kann es sich bei dem Edelstahl um einen ferritischen, korrosionsbeständigen Edelstahl mit der Werkstoffkurzbezeichnung X2CrMoTi18-2 (Werkstoffnummer: 1.4521) handeln. Dieser Edelstahl weist einen Kohlenstoffmassenanteil ≤ 0,025 %, einen Chrommassenanteil von 17,0 % bis 20,0 %, einen Molybdänmassenanteil von 1,80 % bis 2,50 % sowie einen Titanmassenanteil von maximal 0,80 % auf.

Alternativ kann es sich bei dem Edelstahl um einen austenitisch-ferritischen, korrosionsbeständigen Edelstahl mit der Werkstoffkurzbezeichnung X2CrNi22-2 (Werkstoffnummer: 1.4062) handeln. Dieser Edelstahl weist einen Kohlenstoffmassenanteil ≤ 0,03 %, einen Chrommassenanteil von 21,5 % bis 24,0 %, einen Molybdänmassenanteil ≤ 0,45 %, einen Nickelmassenanteil von 1,00 % bis 2,90 % sowie einen Stickstoffmassenanteil von 0,16 % bis 0,28 % auf.

Alternativ kann es sich bei dem Edelstahl um einen austenitisch-ferritischen, korrosionsbeständigen Edelstahl mit der Werkstoffkurzbezeichnung X2CrMnNiN21-5-1 (Werkstoffnummer: 1.4162) handeln. Dieser Edelstahl weist einen Kohlenstoffmassenanteil ≤ 0,04 %, einen Chrommassenanteil von 21,0 % bis 22,0 %, einen Molybdänmassenanteil von 0,10 % bis 0,80 %, einen Nickelmassenanteil von 1,35 % bis 1,70 %, einen Manganmassenanteil von 4,0 % bis 6,0 %, einen Stickstoffmassenanteil von 0,20 % bis 0,25 % sowie einen Kupfermassenanteil von 0,10 % bis 0,80 % auf.

Alternativ kann es sich bei dem Edelstahl um einen austenitisch-ferritischen, korrosionsbeständigen Edelstahl mit der Werkstoffkurzbezeichnung X2CrNiN23-4 (Werkstoffnummer: 1.4362) handeln. Dieser Edelstahl weist einen Kohlenstoffmassenanteil ≤ 0,03 %, einen Chrommassenanteil von 22,0 % bis 24,0 %, einen Molybdänmassenanteil von 0,10 % bis 0,60 %, einen Nickelmassenanteil von 3,5 % bis 5,5 % sowie einen Kupfermassenanteil von 0,10 % bis 0,60 % auf.

Alternativ kann es sich bei dem Edelstahl um einen austenitisch-ferritischen, korrosionsbeständigen Edelstahl mit der Werkstoffkurzbezeichnung X2CrNiMoN22-5-3 (Werkstoffnummer: 1.4462) handeln. Dieser Edelstahl weist einen Kohlenstoffmassenanteil ≤ 0,03 %, einen Chrommassenanteil von 21,0 % bis 23,0 %, einen Molybdänmassenanteil von 2,5 % bis 3,5 %, einen Nickelmassenanteil von 4,5 % bis 6,5 % sowie einen Stickstoffmassenanteil von 0,10 % bis 0,22 % auf.

Alternativ kann es sich bei dem Edelstahl um einen austenitisch-ferritischen, korrosionsbeständigen Edelstahl mit der Werkstoffkurzbezeichnung X2CrNiMnMoCuN24-4-3-2 (Werkstoffnummer: 1.4662) handeln. Dieser Edelstahl weist einen Kohlenstoffmassenanteil ≤ 0,03 %, einen Chrommassenanteil von 23,0 % bis 25,0 %, einen Molybdänmassenanteil von 1,00 % bis 2,00 %, einen Nickelmassenanteil von 3,0 % bis 4,5 %, einen Manganmassenanteil von 2,5 % bis 4,0 % sowie einen Kupfermassenanteil von 0,10 % bis 0,80 % auf.

Alternativ kann es sich bei dem Edelstahl um einen austenitisch-ferritischen, korrosionsbeständigen Edelstahl mit der Werkstoffkurzbezeichnung X2CrNiMoN25-7-4 (Werkstoffnummer: 1.4410) handeln. Dieser Edelstahl weist einen Kohlenstoffmassenanteil ≤ 0,03 %, einen Chrommassenanteil von 24,0 % bis 26,0 %, einen Molybdänmassenanteil von 3,0 % bis 4,5 %, einen Nickelmassenanteil von 6,0 % bis 8,0 % sowie einen Stickstoffmassenanteil von 0,24 % bis 0,35 % auf.

Alternativ kann es sich bei dem Edelstahl um einen austenitisch-ferritischen, korrosionsbeständigen Edelstahl mit der Werkstoffkurzbezeichnung X2CrNiMoCuWN25-7-4 (Werkstoffnummer: 1.4501) handeln. Dieser Edelstahl weist einen Kohlenstoffmassenanteil ≤ 0,03 %, einen Chrommassenanteil von 24,0 % bis 26,0 %, einen Molybdänmassenanteil von 3,0 % bis 4,0 %, einen Nickelmassenanteil von 6,0 % bis 8,0 %, einen Kupfermassenanteil von 0,50 % bis 1,00 %, einen Wolframmassenanteil von 0,50 % bis 1,00 % sowie einen Stickstoffmassenanteil von 0,20 % bis 0,30 % auf.

Alternativ kann es sich bei dem Edelstahl um einen austenitischen, korrosionsbeständigen Edelstahl mit der Werkstoffkurzbezeichnung X2CrNiMo18-15-3 (Werkstoffnummer: 1.4441) handeln. Dieser Edelstahl weist einen Kohlenstoffmassenanteil von maximal 0,030 %, einen Chrommassenanteil von 17,0 % bis 19,0 %, einen Molybdänmassenanteil von 2,70 % bis 3,0 %, einen Nickelmassenanteil von 13,0 % bis 15,0 %, einen Manganmassenanteil von maximal 2,00 %, einen Kupfermassenanteil von maximal 0,50 %, einen Siliziummassenanteil von maximal 0,75 %, einen Phosphormassenanteil von maximal 0,025 %, einen Schwefelmassenanteil von maximal 0,003 % sowie einen Stickstoffmassenanteil von maximal 0,10 % auf.

Alternativ kann es sich bei dem Edelstahl um einen austenitischen, korrosionsbeständigen Edelstahl mit der Werkstoffkurzbezeichnung X5CrNi18-10 (Werkstoffnummer: 1.4301) handeln. Dieser Edelstahl weist einen Kohlenstoffmassenanteil ≤ 0,07 %, einen Chrommassenanteil von 17,5 % bis 19,5 %, einen Nickelmassenanteil von 8,0 % bis 10,5 % sowie einen Stickstoffmassenanteil ≤ 0,11 % auf.

Alternativ kann es sich bei dem Edelstahl um einen austenitischen, korrosionsbeständigen Edelstahl mit der Werkstoffkurzbezeichnung X4CrNi18-12 (Werkstoffnummer: 1.4303) handeln. Dieser Edelstahl weist einen Kohlenstoffmassenanteil ≤ 0,06 %, einen Chrommassenanteil von 17,0 % bis 19,0 %, einen Nickelmassenanteil von 11,0 % bis 13,0 % sowie einen Stickstoffmassenanteil ≤ 0,11 % auf.

Alternativ kann es sich bei dem Edelstahl um einen austenitischen, korrosionsbeständigen Edelstahl mit der Werkstoffkurzbezeichnung X8CrNiS18-9 (Werkstoffnummer: 1.4305) handeln. Dieser Edelstahl weist einen Kohlenstoffmassenanteil ≤ 0,10 %, einen Chrommassenanteil von 17,0 % bis 19,0 %, einen Nickelmassenanteil von 8,0 % bis 10,0 %, einen Schwefelmassenanteil von 0,15 % bis 0,35 % sowie einen Kupfermassenanteil ≤ 1,00 % auf.

Alternativ kann es sich bei dem Edelstahl um einen austenitischen, korrosionsbeständigen Edelstahl mit der Werkstoffkurzbezeichnung X2CrNi19-11 (Werkstoffnummer: 1.4306) handeln. Dieser Edelstahl weist einen Kohlenstoffmassenanteil ≤ 0,030 %, einen Chrommassenanteil von 18,0 % bis 20,0 %, einen Nickelmassenanteil von 10,0 % bis 12,0 % sowie einen Stickstoffmassenanteil ≤ 0,11 % auf.

Alternativ kann es sich bei dem Edelstahl um einen austenitischen, korrosionsbeständigen Edelstahl mit der Werkstoffkurzbezeichnung X2CrNi18-9 (Werkstoffnummer: 1.4307) handeln. Dieser Edelstahl weist einen Kohlenstoffmassenanteil ≤ 0,030 %, einen Chrommassenanteil von 17,5 % bis 19,5 %, einen Nickelmassenanteil von 8,0 % bis 10,5 % sowie einen Stickstoffmassenanteil ≤ 0,11 % auf.

Alternativ kann es sich bei dem Edelstahl um einen austenitischen, korrosionsbeständigen Edelstahl mit der Werkstoffkurzbezeichnung X2CrNi18-10 (Werkstoffnummer: 1.4311) handeln. Dieser Edelstahl weist einen Kohlenstoffmassenanteil ≤ 0,030 %, einen Chrommassenanteil von 17,5 % bis 19,5 %, einen Nickelmassenanteil von 8,5% bis 11,5 % sowie einen Stickstoffmassenanteil von 0,12 % bis 0,22 % auf.

Alternativ kann es sich bei dem Edelstahl um einen austenitischen, korrosionsbeständigen Edelstahl mit der Werkstoffkurzbezeichnung X6CrNiTi18-10 (Werkstoffnummer: 1.4541) handeln. Dieser Edelstahl weist einen Kohlenstoffmassenanteil ≤ 0,08 %, einen Chrommassenanteil von 17,0 % bis 19,0 %, einen Nickelmassenanteil von 9,0 % bis 12,0 % sowie einen Titanmassenanteil von maximal 0,70 % auf.

Alternativ kann es sich bei dem Edelstahl um einen austenitischen, korrosionsbeständigen Edelstahl mit der Werkstoffkurzbezeichnung X6CrNiNb18-10 (Werkstoffnummer: 1.4550) handeln. Dieser Edelstahl weist einen Kohlenstoffmassenanteil ≤ 0,08 %, einen Chrommassenanteil von 17,0 % bis 19,0 %, einen Nickelmassenanteil von 9,0 % bis 12,0 % sowie einen Niobmassenanteil von maximal 1,00 % auf.

Alternativ kann es sich bei dem Edelstahl um einen austenitischen, korrosionsbeständigen Edelstahl mit der Werkstoffkurzbezeichnung X3CrNiCu18-9-4 (Werkstoffnummer: 1.4567) handeln. Dieser Edelstahl weist einen Kohlenstoffmassenanteil ≤ 0,04 %, einen Chrommassenanteil von 17,0 % bis 19,0 %, einen Nickelmassenanteil von 8,5 % bis 10,5 % sowie einen Kupfermassenanteil von 3,0 % bis 4,0 % auf.

Alternativ kann es sich bei dem Edelstahl um einen austenitischen, korrosionsbeständigen Edelstahl mit der Werkstoffkurzbezeichnung X10CrNi18-8 (Werkstoffnummer: 1.4310) handeln. Dieser Edelstahl weist einen Kohlenstoffmassenanteil von 0,05 % bis 0,15 %, einen Chrommassenanteil von 16,0 % bis 19,0 %, einen Molybdänmassenanteil ≤ 0,80 % sowie einen Nickelmassenanteil von 6,0 % bis 9,5 % auf.

Alternativ kann es sich bei dem Edelstahl um einen austenitischen, korrosionsbeständigen Edelstahl mit der Werkstoffkurzbezeichnung X5CrNiMo17-12-2 (Werkstoffnummer: 1.4401) handeln. Dieser Edelstahl weist einen Kohlenstoffmassenanteil ≤ 0,07 %, einen Chrommassenanteil von 16,5 % bis 18,5 %, einen Molybdänmassenanteil von 2,00 % bis 2,50 %, einen Nickelmassenanteil von 10,0 % bis 13,0 % sowie einen Stickstoffmassenanteil ≤ 0,10 % auf.

Alternativ kann es sich bei dem Edelstahl um einen austenitischen, korrosionsbeständigen Edelstahl mit der Werkstoffkurzbezeichnung X2CrNiMo17-12-2 (Werkstoffnummer: 1.4404) handeln. Dieser Edelstahl weist einen Kohlenstoffmassenanteil ≤ 0,030 %, einen Chrommassenanteil von 16,5 % bis 18,5 %, einen Molybdänmassenanteil von 2,00 % bis 2,50 %, einen Nickelmassenanteil von 10,0 % bis 13,0 % sowie einen Stickstoffmassenanteil ≤ 0,10 % auf.

Alternativ kann es sich bei dem Edelstahl um einen austenitischen, korrosionsbeständigen Edelstahl mit der Werkstoffkurzbezeichnung X6CrNiMoTi17-12-2 (Werkstoffnummer: 1.4571) handeln. Dieser Edelstahl weist einen Kohlenstoffmassenanteil ≤ 0,08 %, einen Chrommassenanteil von 16,5 % bis 18,5 %, einen Molybdänmassenanteil von 2,00 % bis 2,50 %, einen Nickelmassenanteil von 10,5 % bis 13,5 % sowie einen Titanmassenanteil von maximal 0,70 % auf.

Alternativ kann es sich bei dem Edelstahl um einen austenitischen, korrosionsbeständigen Edelstahl mit der Werkstoffkurzbezeichnung X2CrNiMoN17-13-3 (Werkstoffnummer: 1.4429) handeln. Dieser Edelstahl weist einen Kohlenstoffmassenanteil ≤ 0,030 %, einen Chrommassenanteil von 16,5 % bis 18,5 %, einen Molybdänmassenanteil von 2,5 % bis 3,0 %, einen Nickelmassenanteil von 11,0 % bis 14,0 % sowie einen Stickstoffmassenanteil von 0,12 % bis 0,22 % auf.

Alternativ kann es sich bei dem Edelstahl um einen austenitischen, korrosionsbeständigen Edelstahl mit der Werkstoffkurzbezeichnung X2CrNiMo18-14-3 (Werkstoffnummer: 1.4435) handeln. Dieser Edelstahl weist einen Kohlenstoffmassenanteil ≤ 0,030 %, einen Chrommassenanteil von 17,0 % bis 19,0 %, einen Molybdänmassenanteil von 2,5 % bis 3,0 %, einen Nickelmassenanteil von 12,5 % bis 15,0 % sowie einen Stickstoffmassenanteil ≤ 0,10 % auf.

Alternativ kann es sich bei dem Edelstahl um einen austenitischen, korrosionsbeständigen Edelstahl mit der Werkstoffkurzbezeichnung X3CrNiMo17-13-3 (Werkstoffnummer: 1.4436) handeln. Dieser Edelstahl weist einen Kohlenstoffmassenanteil ≤ 0,05 %, einen Chrommassenanteil von 16,5 % bis 18,5 %, einen Molybdänmassenanteil von 2,5 % bis 3,0 %, einen Nickelmassenanteil von 10,5 % bis 13,0 % sowie einen Stickstoffmassenanteil ≤ 0,10 % auf.

Alternativ kann es sich bei dem Edelstahl um einen austenitischen, korrosionsbeständigen Edelstahl mit der Werkstoffkurzbezeichnung X2CrNiMoN17-13-5 (Werkstoffnummer: 1.4439) handeln. Dieser Edelstahl weist einen Kohlenstoffmassenanteil ≤ 0,030 %, einen Chrommassenanteil von 16,5 % bis 18,5 %, einen Molybdänmassenanteil von 4,0 % bis 5,0 %, einen Nickelmassenanteil von 12,5 % bis 14,5 % sowie einen Stickstoffmassenanteil von 0,12 % bis 0,22 % auf.

Alternativ kann es sich bei dem Edelstahl um einen austenitischen, korrosionsbeständigen Edelstahlmit der Werkstoffkurzbezeichnung X1NiCrMoCu25-20-5 (Werkstoffnummer: 1.4539) handeln. Dieser Edelstahl weist einen Kohlenstoffmassenanteil ≤ 0,020 %, einen Chrommassenanteil von 19,0 % bis 21,0 %, einen Molybdänmassenanteil von 4,0 % bis 5,0 %, einen Nickelmassenanteil von 24,0 % bis 26,0 %, einen Kupfermassenanteil von 1,20 % bis 2,00 % sowie einen Stickstoffmassenanteil ≤ 0,15 % auf.

Alternativ kann es sich bei dem Edelstahl um einen austenitischen, korrosionsbeständigen Edelstahl mit der Werkstoffkurzbezeichnung X2CrNiMnMoNbN25-18-5-4 (Werkstoffnummer: 1.4565) handeln. Dieser Edelstahl weist einen Kohlenstoffmassenanteil ≤ 0,030 %, einen Chrommassenanteil von 24,0 % bis 26,0 %, einen Molybdänmassenanteil von 4,0 % bis 5,0 %, einen Nickelmassenanteil von 16,0 % bis 19,0 %, einen Manganmassenanteil von 5,0 % bis 7,0 %, einen Stickstoffmassenanteil von 0,30 % bis 0,60 % sowie einen Niobmassenanteil ≤ 0,15 % auf.

Alternativ kann es sich bei dem Edelstahl um einen austenitischen, korrosionsbeständigen Edelstahl mit der Werkstoffkurzbezeichnung X1NiCrMoCuN25-20-7 (Werkstoffnummer: 1.4529) handeln. Dieser Edelstahl weist einen Kohlenstoffmassenanteil ≤ 0,020 %, einen Chrommassenanteil von 19,0 % bis 21,0 %, einen Molybdänmassenanteil von 6,0 % bis 7,0 %, einen Nickelmassenanteil von 24,0 % bis 26,0 %, einen Kupfermassenanteil von 0,50 % bis 1,50 % sowie einen Stickstoffmassenanteil von 0,15 % bis 0,25 % auf.

Alternativ kann es sich bei dem Edelstahl um einen austenitischen, korrosionsbeständigen Edelstahl mit der Werkstoffkurzbezeichnung X1CrNiMoCuN20-18-7 (Werkstoffnummer: 1.4547) handeln. Dieser Edelstahl weist einen Kohlenstoffmassenanteil ≤ 0,020 %, einen Chrommassenanteil von 19,5 % bis 20,5 %, einen Molybdänmassenanteil von 6,0 % bis 7,0 %, einen Nickelmassenanteil von 17,5 % bis 18,5 %, einen Kupfermassenanteil von 0,50 % bis 1,00 % sowie einen Stickstoffmassenanteil von 0,18 % bis 0,25 % auf.

Alternativ kann es sich bei dem Edelstahl um einen austenitischen, korrosionsbeständigen Edelstahl mit der Werkstoffkurzbezeichnung X1CrNiMoCuN24-22-8 (Werkstoffnummer: 1.4652) handeln. Dieser Edelstahl weist einen Kohlenstoffmassenanteil ≤ 0,020 %, einen Chrommassenanteil von 23,0 % bis 25,0 %, einen Molybdänmassenanteil von 7,0 % bis 8,0 %, einen Nickelmassenanteil von 21,0 % bis 23,0 %, einen Manganmassenanteil von 2,0 % bis 4,0 % sowie einen Stickstoffmassenanteil von 0,45 % bis 0,55 % auf.

In weiterer Ausgestaltung der Erfindung handelt es sich bei dem medizintechnischen Produkt um ein medizinisches, vorzugsweise chirurgisches, Instrument. Bei dem Instrument kann es sich weiter um ein wiederverwendbares Instrument oder um ein Einweginstrument ("single use instrument") handeln.

Ferner kann es sich bei dem Instrument um ein minimalinvasives Instrument, d.h. um ein in der minimalinvasiven Chirurgie einsetzbares Instrument, handeln.

Das chirurgische Instrument kann insbesondere aus der Gruppe bestehend aus spreizendes Instrument, fassendes Instrument, klemmendes Instrument, schneidendes Instrument, Nahtgerät, Endoskop und kombiniertes Instrument ausgewählt sein.

Bei dem spreizenden Instrument kann es sich beispielsweise um einen Wundhaken, einen Retraktor, einen Wundspreizer, einen Brustbeinspreizer, einen Wundsperrer, ein Spekulum oder eine Trokarhülse handeln.

Bei dem fassenden Instrument kann es sich beispielsweise um eine Pinzette, eine Klemme, einen Nadelhalter oder eine Fasszange handeln.

Bei dem klemmenden Instrument kann es sich beispielsweise um eine weiche Klemme, insbesondere zum temporären Abklemmen von Darm und feinen Gefäßen, oder um eine Präparierklemme handeln.

Bei dem schneidenden Instrument kann es sich beispielsweise um ein Skalpell, ein Messer, eine Schere, eine Branchenzange, eine Knochensplitterzange, eine Ringzange, ein Elektrotom, ein Konchotom, einen Kauter oder ein Ultraschallmesser handeln.

Bei dem Nahtgerät kann es sich insbesondere um ein Klammernahtgerät (Stapler) oder um einen Klammerentferner handeln.

Bei dem kombinierten Instrument kann es sich um einen Endostapler oder ein Klammernahtgerät, welches beispielsweise ein Hohlorgan verklammert und zugleich präzise schneidet, handeln. Weiterhin kann es sich bei dem kombinierten Instrument um einen kombinierten Nadelhalter, der als Universalnahtgerät sowohl fassen als auch schneiden kann, handeln.

Ferner kann es sich bei dem chirurgischen Instrument um einen Hammer handeln.

Ferner kann es sich bei dem chirurgischen Instrument um einen Meißel, insbesondere Flachoder Hohlmeißel wie Knochenhohlmeißel, oder um eine Kürette, insbesondere Knochenkürette, handeln.

Ferner kann es sich bei dem chirurgischen Instrument um eine Sonde handeln.

Ferner kann es sich bei dem chirurgischen Instrument um eine Knochenstanze handeln.

Ferner kann es sich bei dem chirurgischen Instrument um einen Hebel oder Elevator oder ein Raspatorium handeln.

Gemäß einem zweiten Aspekt betrifft die Erfindung ein medizintechnisches Produkt, aufweisend oder bestehend aus einem Metall oder einer Legierung, wobei das medizintechnische Produkt hergestellt oder herstellbar ist nach einem Verfahren gemäß erstem Erfindungsaspekt und folgendes Merkmal aufweist:
- eine Passivschicht, insbesondere aus Chromoxid, mit einer Dicke von 1 nm bis 10 nm, insbesondere 3 nm bis 10 nm, bevorzugt 5 nm bis 10 nm, welche die Oberfläche des medizintechnischen Produkts wenigstens abschnittsweise, insbesondere nur abschnittsweise oder vollständig, beschichtet.

Das medizintechnische Produkt kann ein Lochkorrosionspotential von 100 mV bis 1200 mV, insbesondere von 200 mV bis 800 mV, vorzugsweise von 400 mV bis 500 mV (gemessen gegen eine Standardwasserstoffelektrode) aufweisen.

Das medizintechnische Produkt kann einen Kontaktwinkel von 80 ° bis 140 °, insbesondere von 90 ° bis 130 °, vorzugsweise von 100 ° bis 120 ° aufweisen.

Die vorgenannten Lochkorrosionspotentiale sowie Kontaktwinkel sind im Hinblick auf die Korrosionsbeständigkeit des medizintechnischen Produkts besonders vorteilhaft.

Unter dem Ausdruck "Lochkorrosionspotential" soll im Sinne der vorliegenden Erfindung das elektrochemische Potential verstanden werden, das mit einer elektrochemischen Zelle unter Verwendung einer Drei-Elektroden-Anordnung bestimmt werden kann. Das Lochkorrosionspotential ist durch einen schnellen Stromanstieg charakterisiert und beschreibt den Zusammenbruch der Passivschicht unter einsetzender Lochkorrosion. Eine Erhöhung des Lochkorrosionspotentials bewirkt eine Verbesserung der Korrosionsbeständigkeit durch eine Reduzierung der Lochkorrosionsanfälligkeit. Die Messung des Lochkorrosionspotentials kann gemäß ASTM G5-13-1 oder DIN EN ISO 10993-15 erfolgen.

Unter dem Ausdruck "Kontaktwinkel" soll im Sinne der vorliegenden Erfindung der Winkel verstanden werden, welchen ein Flüssigkeitstropfen auf der Oberfläche des medizintechnischen Produkts zu dessen Oberfläche bildet. Ein verringerter Kontaktwinkel geht mit einem verringerten Kontakt des Flüssigkeitstropfens an der Oberfläche des medizintechnischen Produkts einher. Eine Verringerung des Kontaktwinkels bewirkt mit besonderem Vorteil eine Verbesserung der Korrosionsbeständigkeit sowie Reinigbarkeit des medizintechnischen Produkts.

Die Messung des Kontaktwinkels kann gemäß ASTM D 7334-08 erfolgen. Alternativ kann die Messung des Kontaktwinkels mittels eines Kontaktwinkelmessgeräts der Firma dataPhysics (Contact Angle System OCA 15 Plus) und unter Verwendung einer 0,9 %igen NatriumchloridLösung (B.Braun) durchgeführt werden, wobei das Tropfenvolumen 1 µl beträgt. Zur Messung des Kontaktwinkels können die Proben in diesem Fall in einem regulären Fertigungsprozess ausgewaschen und vor der Messung in vollentsalztem Wasser im Ultraschallbad für 5 Minuten gereinigt werden, wobei die Proben direkt vor der Messung mit vollentsalztem Wasser abgewaschen und mit ölfreier Druckluft abgeblasen werden.

Bei dem medizintechnischen Produkt handelt es sich vorzugsweise um ein medizinisches, bevorzugt chirurgisches, Instrument.

Bezüglich weiterer Merkmale und Vorteile des medizintechnischen Produkts wird zur Vermeidung von Wiederholungen vollständig auf die im Rahmen des ersten Erfindungsaspekts gemachten Ausführungen Bezug genommen. Die dort in Bezug auf das Verfahren sowie das medizintechnische Produkt beschriebenen Merkmale und Vorteile gelten sinngemäß auch für das medizintechnische Produkt gemäß zweitem Erfindungsaspekt.

Weitere Merkmale und Vorteile der Erfindung ergeben sich aus den Ansprüchen sowie aus der nachfolgenden Beschreibung bevorzugter Ausführungsformen anhand von Beispielen. Dabei können Merkmale der Erfindung jeweils für sich alleine oder in Kombination miteinander verwirklicht sein. Die nachfolgend beschriebenen Ausführungsformen dienen der weiteren Erläuterung der Erfindung, ohne diese hierauf zu beschränken.

### BEISPIELTEIL

### 1. Oberflächenbehandlung eines chirurgischen Instruments bzw. repräsentativer Probekörper dessen nach einem erfindungsgemäßen Verfahren

Die verwendeten Probekörper wurden aus dem identischen Material (X20Cr13) und mit den identischen Fertigungsschritten wie die chirurgischen Instrumente (z.B. Klemmen, Nadelhalter, Scheren mit Hartmetall und dergleichen) hergestellt.

REM/EDX-Analysen (Fremdmaterial und Materialdopplungen) wurden an den Instrumenten durchgeführt.

Potentiodynamische Prüfungen (Lochkorrosionspotential) wurden ebenfalls an den Instrumenten durchgeführt.

Kontaktwinkelmessungen (Kontaktwinkel) wurden an Probeplättchen (plane Fläche ohne Abschattungen) durchgeführt.

Kratzbeständigkeitsmessungen (Volumen der Verschleißspur) wurde mittels eines Pin-on-Disc Versuchs an Probeplättchen durchgeführt.

Vor der Oberflächenbehandlung wurden chirurgische Instrumente und Probenplättchen nach der aktuellen Herstellungskette chirurgischer Instrumente in Form gebracht und wärmebehandelt.

Zur anschließenden Oberflächenbehandlung wurden ein chirurgisches Instrument (Aesculap Klemme BH110R) und Probeplättchen zunächst während eines Zeitraumes von vier Stunden mittels Gleitschleifen in saurer Lösung behandelt und anschließend durch Gleitschleifen in wässriger Lösung über einen Zeitraum von einer Stunde aufgehellt.

Danach wurden das chirurgische Instrument und die Probeplättchen mittels Strahlen behandelt. Als Strahlmittel wurden Edelstahldrahtkorn mit einem mittleren Kugeldurchmesser von 300 µm verwendet. Für das Strahlen wurde eine Injektorstrahlanlage verwendet. Das Strahlen wurde unter einem Druck von 2 bar durchgeführt.

Anschließend wurde die Oberfläche des chirurgischen Instruments und der Probeplättchen in einem Elektrolyt aus Phosphorsäure (38 Gew.-% bis 58 Gew.-%) und Schwefelsäure (26,6 Gew.- % bis 52,0 Gew.-%) bei 80°C elektrochemisch bearbeitet. Hierzu wurde zuerst mit einer Stromdichte von 18,5 A/dm² gearbeitet. Das Elektropolieren wurde während eines Zeitraums von 90 Sekunden durchgeführt. Hierbei hat sich eine Spannung von 5 bis 6 Volt eingestellt. Im Anschluss wurde mit einer Stromdichte von 1,9 A/dm² weiter gearbeitet. Das elektrochemische Ätzen würde während eines Zeitraums von 90 Sekunden durchgeführt. Hierbei hat sich eine Spannung von 1,8 bis 2,1 Volt eingestellt. Zum Schluss wurde nochmals mit einer Stromdichte von 18,5 A/dm² gearbeitet. Das Elektropolieren wurde während eines Zeitraums von 10 Sekunden durchgeführt. Hierbei hat sich eine Spannung von 5 bis 6 Volt eingestellt.

Im Anschluss daran wurde die Oberfläche des chirurgischen Instruments und der Probekörper passiviert. Hierzu wurden das chirurgische Instrument und die Probeplättchen in eine 14,4 Gew.- %ige Salpetersäure eingetaucht. Das Passivieren wurde während eines Zeitraumes von 10 Minuten bei einer Temperatur von 40 °C durchgeführt.

Zum Schluss wurden das Instrument und die Probeplättchen bei 80 °C für 10 Minuten unter Luftatmosphäre getrocknet.

Nach Abschluss der Oberflächenbehandlung des chirurgischen Instruments und der Probekörper waren keine Materialdopplungen bzw. Materialüberlappungen sowie kein Fremdmaterialübertrag feststellbar. Die Instrumente wiesen ein Lochkorrosionspotential von 565 mV auf. Die Probeplättchen hatten einen Kontaktwinkel von 106,8 °. Das Volumen der Verschleißspur war nicht messbar, da der Verschleiß zu gering war.

### 2. Oberflächenbehandlung eines chirurgischen Instruments nach einem nicht erfindungsgemäßen Verfahren

Ein chirurgisches Instrument (Aesculap Klemme BH110R) und Probeplättchen wurden zunächst während eines Zeitraumes von vier Stunden mittels Gleitschleifen behandelt. Danach ließ man das chirurgische Instrument und die Probeplättchen während eines Zeitraumes von einer Stunde aufhellen.

Danach wurden das chirurgische Instrument und die Probekörper mittels Strahlen behandelt. Hierzu wurden Glasperlen mit einem mittleren Durchmesser von 40 µm bis 70 µm verwendet. Das Strahlen wurde in einer Injektorstrahlanlage unter einem Druck von 4 bar durchgeführt.

Anschließend wurden das chirurgische Instrument und die Probekörper einer Passivierung unterworfen. Hierzu wurde eine 10 Gew.-%ige Zitronensäurelösung verwendet. Das Passivieren erfolgte während eines Zeitraumes von 10 Minuten bei einer Temperatur von 55 °C.

Nach Abschluss der Oberflächenbehandlung des chirurgischen Instruments und der Probeplättchen waren viele Materialdopplungen bzw. Materialüberlappungen feststellbar. Es konnte zudem ein Fremdmaterialübertrag von 1,2 % nachgewiesen werden. Das Lochkorrosionspotential der Korrosionsprobekörper betrug 356 mV. Außerdem wiesen die Probeplättchen einen Kontaktwinkel von 66,0 ° auf. Der Volumenabtrag in der Verschleißspur lag bei 55244 µm³.

### 3. Fazit

Die oben beschriebene Gegenüberstellung eines erfindungsgemäßen Verfahrens und eines gattungsgemäßen Verfahrens zeigt, dass das erfindungsgemäße Verfahren zu korrosions- und insbesondere kratzbeständigeren Produkten führt. Obendrein ist das erfindungsgemäße Verfahren dazu geeignet, das Risiko des Auftretens von Oberflächenverfärbungen gegenüber gattungsgemäßen Verfahren zu verringern. Ferner führt das erfindungsgemäße Verfahren zu besser reinigbaren Produkten (siehe gemessene Kontaktwinkel).

## Patentansprüche

1. Verfahren zum Oberflächenbehandeln und/oder Herstellen eines medizintechnischen Produkts, wobei das medizintechnische Produkt ein Metall oder eine Legierung aufweist oder aus einem Metall oder einer Legierung besteht, **dadurch gekennzeichnet, dass** das Verfahren die nachfolgenden Schritte aufweist:
a) Mattieren einer Oberfläche des medizintechnischen Produkts,
b) Elektropolieren der mattierten Oberfläche des medizintechnischen Produkts,
c) elektrochemisches Ätzen der mattierten und elektropolierten Oberfläche des medizintechnischen Produkts und
d) Elektropolieren der mattierten, elektropolierten und elektrochemisch geätzten Oberfläche des medizintechnischen Produkts,
wobei der Schritt b) und der Schritt d) jeweils bei einer Spannung von 2,0 V bis 10,0 V und/oder jeweils bei einer Stromdichte von 5 A/dm² bis 50 A/dm² und der Schritt c) bei einer Spannung von 1,2 V bis ≤ 2,1 V und/oder bei einer Stromdichte von 1,4 A/dm² bis 2,4 A/dm² durchgeführt werden.

2. Verfahren nach Anspruch 1, **dadurch gekennzeichnet, dass** vor dem Durchführen von Schritt a) ein Schleifen, vorzugsweise Gleit- und/oder Bandschleifen, der Oberfläche des medizintechnischen Produkts durchgeführt wird.

3. Verfahren nach Anspruch 1 oder 2, **dadurch gekennzeichnet, dass** zum Durchführen von Schritt a) die Oberfläche des medizintechnischen Produkts mit einem Strahlmittel, insbesondere duktilen und/oder ecken- und/oder kantenfrei gestalteten Strahlmittel, insbesondere aufweisend oder bestehend aus einem Metall oder einer Legierung, behandelt wird.

4. Verfahren nach einem der vorhergehenden Ansprüche, **dadurch gekennzeichnet, dass** der Schritt b) während eines Zeitraumes von 30 s bis 130 s, insbesondere 60 s bis 100 s, vorzugsweise 90 s, durchgeführt wird.

5. Verfahren nach einem der vorhergehenden Ansprüche, **dadurch gekennzeichnet, dass** der Schritt b) und der Schritt d) jeweils bei einer Spannung von 5 V bis 9 V, besonders bevorzugt von 7 V bis 8 V, und/oder jeweils bei einer Stromdichte von 8 A/dm² bis 40 A/dm², vorzugsweise 15 A/dm² bis 20 A/dm², durchgeführt wird.

6. Verfahren nach einem der vorhergehenden Ansprüche, **dadurch gekennzeichnet, dass** der Schritt c) während eines Zeitraumes von 30 s bis 130 s, insbesondere 70 s bis 110 s, vorzugsweise 100 s, durchgeführt wird.

7. Verfahren nach einem der vorhergehenden Ansprüche, **dadurch gekennzeichnet, dass** der Schritt c) bei einer an der Anode anliegenden Spannung von 1,4 V bis 1,7 V, besonders bevorzugt 1,45 V bis 1,65 V, und/oder bei einer Stromdichte von 1,6 A/dm² bis 2,2 A/dm², vorzugsweise 1,8 A/dm² bis 2,0 A/dm², durchgeführt wird.

8. Verfahren nach einem der vorhergehenden Ansprüche, **dadurch gekennzeichnet, dass** der Schritt d) während eines kürzeren Zeitraumes durchgeführt wird als der Schritt b), bevorzugt während eines Zeitraumes von 5 s bis 20 s, insbesondere 7 s bis 15 s, vorzugsweise 10 s.

9. Verfahren nach einem der vorhergehenden Ansprüche, **dadurch gekennzeichnet, dass** zum Durchführen der Schritte b), c) und d) jeweils eine saure, wässrige Elektrolytlösung, insbesondere aufweisend eine Mineralsäure, insbesondere ausgewählt aus der Gruppe bestehend aus Phosphorsäure, Schwefelsäure und ein Gemisch davon, verwendet wird.

10. Verfahren nach einem der vorhergehenden Ansprüche, **dadurch gekennzeichnet, dass** nach dem Durchführen von Schritt d) ein Schritt e) Behandeln der mattierten, elektropolierten, elektrochemisch geätzten und nochmals elektropolierten Oberfläche des medizintechnischen Produkts mit einer Passiviersäure oder einer passiviersäurehaltigen Lösung durchgeführt wird.

11. Verfahren nach Anspruch 10, **dadurch gekennzeichnet, dass** als Passiviersäure Zitronen- und/oder Salpetersäure verwendet wird.

12. Verfahren nach einem der vorhergehenden Ansprüche, **dadurch gekennzeichnet, dass** nach dem Durchführen von Schritt e) ein Schritt f) Verpacken des medizintechnischen Produkts und zwischen dem Schritt e) und dem Schritt f) ein Schritt ef) Sterilisieren des medizintechnischen Produkts oder nach dem Durchführen von Schritt f) ein Schritt g) Sterilisieren des medizintechnischen Produkts durchgeführt wird.

13. Verfahren nach einem der vorhergehenden Ansprüche, **dadurch gekennzeichnet, dass** das medizintechnische Produkt aus einem Edelstahl, insbesondere chromhaltigen Edelstahl, bevorzugt chromhaltigen, korrosionsbeständigen Edelstahl, insbesondere martensitischen, korrosionsbeständigen Edelstahl, besteht.

14. Medizintechnisches Produkt, aufweisend oder bestehend aus einem Metall oder einer Legierung, hergestellt oder herstellbar nach einem Verfahren nach einem der vorhergehenden Ansprüche und aufweisend
- eine Passivschicht mit einer Dicke von 1 nm bis 10 nm, welche die Oberfläche des medizintechnischen Produkts wenigstens abschnittsweise beschichtet.

15. Medizintechnisches Produkt nach Anspruch 14, **dadurch gekennzeichnet, dass** es sich bei dem medizintechnischen Produkt um ein medizinisches, insbesondere chirurgisches, Instrument handelt.

## Claims

1. Method of surface treatment and/or manufacture of a medical product, wherein the medical product includes a metal or an alloy or consists of a metal or an alloy, **characterized in that** the method comprises the following steps:
a) dulling a surface of the medical product,
b) electropolishing the dulled surface of the medical product,
c) electrochemically etching the dulled and electropolished surface of the medical product and
d) electropolishing the dulled, electropolished and electrochemically etched surface of the medical product,
wherein step b) and step d) are each conducted at a voltage of 2.0 V to 10.0 V and/or each at a current density of 5 A/dm² to 50 A/dm² and step c) at a voltage of 1.2 V to ≤ 2.1 V and/or at a current density of 1.4 A/dm² to 2.4 A/dm².

2. Method according to Claim 1, **characterized in that** the performance of step a) is preceded by a grinding operation, preferably slide finishing and/or belt finishing, on the surface of the medical product.

3. Method according to Claim 1 or 2, **characterized in that** step a) is performed by treating the surface of the medical product with a blasting agent, especially ductile blasting agents and/or blasting agents that are free of corners and/or edges, especially comprising or consisting of a metal or an alloy.

4. Method according to any of the preceding claims, **characterized in that** step b) is conducted over a period of 30 s to 130 s, especially 60 s to 100 s, preferably 90 s.

5. Method according to any of the preceding claims, **characterized in that** step b) and step d) are each conducted at a voltage of 5 V to 9 V, more preferably of 7 V to 8 V, and/or each at a current density of 8 A/dm² to 40 A/dm², preferably 15 A/dm² to 20 A/dm².

6. Method according to any of the preceding claims, **characterized in that** step c) is conducted over a period of 30 s to 130 s, especially 70 s to 110 s, preferably 100 s.

7. Method according to any of the preceding claims, **characterized in that** step c) is conducted at a voltage applied to the anode of 1.4 V to 1.7 V, more preferably of 1.45 V to 1.65 V, and/or at a current density of 1.6 A/dm² to 2.2 A/dm², preferably 1.8 A/dm² to 2.0 A/dm².

8. Method according to any of the preceding claims, **characterized in that** step d) is conducted over a shorter period than step b), preferably over a period of 5 s to 20 s, especially 7 s to 15 s, preferably 10 s.

9. Method according to any of the preceding claims, **characterized in that** steps b), c) and d) are each conducted using an acidic aqueous electrolyte solution, especially comprising a mineral acid, especially selected from the group consisting of phosphoric acid, sulfuric acid and a mixture thereof.

10. Method according to any of the preceding claims, **characterized in that** the performance of step d) is followed by performance of a step e) of treating the dulled, electropolished, electrochemically etched and once again electropolished surface of the medical product with a passivating acid or a passivating acid-containing solution.

11. Method according to Claim 10, **characterized in that** the passivating acid used is citric acid and/or nitric acid.

12. Method according to any of the preceding claims, **characterized in that** the performance of step e) is followed by performance of a step f) of packing the medical product and a step ef) of sterilizing the medical product is conducted between step e) and step f) or the performance of step f) is followed by performance of a step g) of sterilizing the medical product.

13. Method according to any of the preceding claims, **characterized in that** the medical product consists of a stainless steel, especially chromiumcontaining stainless steel, preferably chromiumcontaining corrosion-resistant stainless steel, especially martensitic corrosion-resistant stainless steel.

14. Medical product comprising or consisting of a metal or an alloy, produced or producible by a method according to any of the preceding claims and comprising
- a passive layer having a thickness of 1 nm to 10 nm that coats at least parts of the surface of the medical product.

15. Medical product according to Claim 14, **characterized in that** the medical product is a medical, especially surgical, instrument.

## Revendications

1. Procédé de traitement thermique et/ou de fabrication d'un dispositif médico-technique, le dispositif médico-technique comportant un métal ou un alliage ou étant constitué d'un métal ou d'un alliage, **caractérisé en ce que** le procédé comprend les étapes suivantes :
a) matage d'une surface du dispositif médico-technique,
b) électropolissage de la surface matée du dispositif médico-technique,
c) gravure électrochimique de la surface matée et électropolie du dispositif médico-technique et
d) électropolissage de la surface matée, électropolie et ayant subi une gravure électrochimique, du dispositif médico-technique,
dans lequel l'étape b) et l'étape d) sont mises en œuvre chacune sous une tension de 2,0 V à 10,0 V, et/ou chacune pour une densité de courant de 5 A/dm² à 50 A/dm² et l'étape c) est mise en œuvre sous une tension de 1,2 V à ≤ 2,1 V et/ou pour une densité de courant de 1,4 A/dm² à 2,4 A/dm².

2. Procédé selon la revendication 1, **caractérisé en ce que**, avant mise en œuvre de l'étape a), on met en œuvre un travail par abrasion, de préférence un ponçage ou un travail à la bande abrasive, de la surface du dispositif médico-technique.

3. Procédé selon la revendication 1 ou 2, **caractérisé en ce que**, pour la mise en œuvre de l'étape a), on traite la surface du dispositif médico-technique avec un agent de grenaillage, en particulier un agent de grenaillage ductile et/ou sans angles et/ou sans arêtes, comportant de préférence un métal ou un alliage ou en en étant constitué.

4. Procédé selon l'une des revendications précédentes, **caractérisé en ce que** l'étape b) est mise en œuvre pendant une durée de 30 secondes à 130 secondes, en particulier de 60 secondes à 100 secondes, de préférence de 90 secondes.

5. Procédé selon l'une des revendications précédentes, **caractérisé en ce que** l'étape b) et l'étape d) sont chacune mises en œuvre sous une tension de 5 V à 9 V, d'une manière particulièrement préférée de 7 V à 8 V, et/ou chacune pour une densité de courant de 8 A/dm² à 40 A/dm², de préférence de 15 A/dm² à 20 A/dm².

6. Procédé selon l'une des revendications précédentes, **caractérisé en ce que** l'étape c) est mise en œuvre pendant une durée de 30 secondes à 130 secondes, en particulier de 70 secondes à 110 secondes, de préférence de 100 secondes.

7. Procédé selon l'une des revendications précédentes, **caractérisé en ce que** l'étape c) est mise en œuvre sous une tension appliquée à l'anode de 1,4 V à 1,7 V, d'une manière particulièrement préférée de 1,45 V à 1,65 V, et/ou pour une densité de courant de 1,6 A/dm² à 2,2 A/dm2, de préférence de 1,8 A/dm² à 2,0 A/dm².

8. Procédé selon l'une des revendications précédentes, **caractérisé en ce que** l'étape d) est mise en œuvre pendant une durée plus courte que l'étape b), de préférence pendant une durée de 5 secondes à 20 secondes, en particulier de 7 secondes à 15 secondes, de préférence de 10 secondes.

9. Procédé selon l'une des revendications précédentes, **caractérisé en ce que**, pour mettre en œuvre les étapes b), c) et d), on utilise pour chacune une solution électrolytique aqueuse acide, comportant de préférence un acide minéral, en particulier choisi dans le groupe consistant en l'acide phosphorique, l'acide sulfurique et un mélange de ceux-ci.

10. Procédé selon l'une des revendications précédentes, **caractérisé en ce que**, après la mise en œuvre de l'étape d), on met en œuvre une étape e) traitement de la surface matée, électropolie, ayant subi une gravure électrochimique et une fois encore électropolie du dispositif médico-technique avec un acide de passivation ou une solution contenant un acide de passivation.

11. Procédé selon la revendication 10, **caractérisé en ce qu'**on utilise comme acide de passivation l'acide citrique et/ou l'acide nitrique.

12. Procédé selon l'une des revendications précédentes, **caractérisé en ce qu'**on met en œuvre, après mise en œuvre de l'étape e) une étape f) conditionnement du dispositif médico-technique et, entre l'étape e) et l'étape f), une étape ef) stérilisation du dispositif médico-technique, ou après la mise en œuvre de l'étape f) une étape g) stérilisation du dispositif médico-technique.

13. Procédé selon l'une des revendications précédentes, **caractérisé en ce que** le dispositif médico-technique est constitué d'un acier inoxydable, en particulier d'un acier inoxydable au chrome, de préférence d'un acier inoxydable au chrome anti-corrosion, en particulier d'un acier inoxydable martensinique anti-corrosion.

14. Dispositif médico-technique comportant un métal ou un alliage, ou en étant constitué, fabriqué ou pouvant être fabriqué par un procédé selon l'une des revendications précédentes et comportant
- une couche de passivation ayant une épaisseur de 1 nm à 10 nm, qui recouvre au moins par segments la surface du dispositif médico-technique.

15. Dispositif médico-technique selon la revendication 14, **caractérisé en ce que**, pour ce qui concerne le dispositif médico-technique, il s'agit d'un instrument médical, en particulier chirurgical.
